# EUROPEAN PATENT SPECIFICATION

(11) **EP 2 967 590 B1**
(45) Date of publication and mention of the grant of the patent: **06.05.2020**
(21) Application number: 14763134.5
(22) Date of filing: 14.03.2014
(51) Int. Cl.: A61B 46/10, A61B 17/10, A61B 10/02, A61B 17/16

(54) **CONTAINMENT ASSEMBLIES**
EINDÄMMUNGSANORDNUNGEN
ENSEMBLES DE CONFINEMENT

(30) Priority: 15.03.2013 US 201313836019
(43) Date of publication of application: 20.01.2016
(73) Proprietor: Teleflex Medical Devices S.à r.l., 2220 Luxembourg, Luxembourg (LU)
(72) Inventor: YOON, Michel, Shavano Park, TX 78249 (US)
(74) Representative: Nguyen-Van-Yen, Christian
(86) International application number: PCT/US2014/029299
(87) International publication number: WO 2014/144757

(56) References cited:
- WO-A1-98/02107
- US-A1- 2006 052 790
- US-A1- 2007 078 346
- US-A1- 2008 045 965
- US-A1- 2008 045 965
- US-A1- 2009 093 677
- US-A1- 2009 194 446
- US-A1- 2011 087 106
- US-A1- 2012 209 317
- US-B1- 6 716 215

## Description

### BACKGROUND

### 1. Field of the Invention

The present invention relates generally to containment assemblies (e.g., bags, casings, and couplers), and more particularly, but not by way of limitation, to containment assemblies configured to receive and/or seal a driver (e.g., a manual and powered driver).

### 2. Description of Related Art

Examples of containment assemblies are shown in, for example, U.S. Patent Application Pub. No. 2009/0194446 and in U.S. Patent No. 7,850,620, both of which are currently co-owned by the owner of this patent application. US 2008/045965 A1 discloses a containment assembly for a driver, the containment assembly comprising a coupler having a first end configured to be coupled to an intraosseous needle set and a second end configured to be coupled to a driver such that the driver can move the intraosseous needle set; and a flexible containment bag having a front portion coupled to the coupler.US 2012/209317 A1 discloses a substantially rigid container for a power driven vessel closure system.

### SUMMARY

The invention is defined by claim 1. Preferred embodiments of the invention are defined by the dependent claims. This disclosure includes embodiments of containment assemblies configured to receive and/or seal a driver (e.g., a manual and powered driver) and that may also be configured to, for example, permit coupling (e.g., directly or indirectly) of the sealed drivers to intraosseous needle sets, as well as related methods and kits. Embodiments of the present containment assemblies, kits, and methods may be useful in procedures such as those that establish access to an intraosseous (IO) space, bone marrow biopsies, and craniotomies, to name a few.

Some embodiments of the present containment assemblies comprise an improved containment assembly for a driver, the containment assembly including a coupler having a first end configured to be coupled to an intraosseous needle set and a second end configured to be coupled to a driver such that the driver can move the intraosseous needle set, the improvement of the containment assembly comprising a containment bag having an interior and: a first end having a first opening and coupled to the coupler such that the coupler covers the first opening; a second end spaced apart from a second opening sized to receive a driver; a flap between the second end and the second opening; and a rod couplable to the flap such that the second opening can be closed and sealed by wrapping at least a portion of the containment bag around the rod. In some embodiments, the containment bag is substantially sterile. In some embodiments, the rod is coupled to the containment bag. In some embodiments, at least one outer portion of the rod comprises a circular cross-section and is configured to be bent to prevent the second end of the containment bag from moving away from the first end of the containment bag after the second opening is closed. In some embodiments, the containment assemblies comprise a ring disposed over the first end of the containment bag to prevent the containment bag from decoupling from the coupler.

Some embodiments of the present containment assemblies comprise an improved containment assembly for a driver, the containment assembly including a coupler having a first end configured to be coupled to an intraosseous needle set and a second end configured to be coupled to a driver such that the driver can move the intraosseous needle set, the improvement of the containment assembly comprising a containment bag having an interior and: a first end having a first opening, the first end coupled to the coupler such that the coupler covers the first opening; a second end having a second opening, a first lockable track adjacent the second opening, and a second lockable track adjacent to the second opening; and a tab movably coupled to the first and second lockable tracks and configured to be moved in a first direction to cause the first lockable track to engage the second lockable track to close the second opening and seal the interior. In some embodiments, the containment bag is substantially sterile.

Some embodiments of the present containment assemblies comprise an improved containment assembly for a driver, the containment assembly including a coupler having a first end configured to be coupled to an intraosseous needle set and a second end configured to be coupled to a driver such that the driver can move the intraosseous needle set, the improvement of the containment assembly comprising a containment bag having an interior and: a first end having a first opening, the first end coupled to the coupler such that the coupler covers the first opening; a second end having a second opening; and at least one drawstring coupled to the second end of the containment bag, the at least one drawstring pullable to cinch the containment bag to close the second opening and seal the interior. In some embodiments, the containment bag is in a folded configuration and the second end is configured to be pulled away from the first end to move the containment bag to an unfolded configuration. In some embodiments, the containment bag is configured such that the drawstring is pullable to move the containment bag from the folded configuration to the unfolded configuration. In some embodiments, the containment bag has a plurality of folds between the first end and the second end. In some embodiments, the containment bag is substantially sterile. In some embodiments, the containment assemblies further comprise at least one retaining strip configured to be coupled to the containment bag to maintain the containment bag in the folded position until a user pulls the second end away from the first end to move the containment bag to the unfolded configuration. In some embodiments, the at least one retaining strip is configured to decouple from at least one of the first and second ends of the containment bag when a sufficient force is applied to move the containment bag from the folded configuration to the unfolded configuration. In some embodiments, the containment bag can move into the unfolded configuration when the retaining strip is decoupled from one of first and second ends of the containment bag. In some embodiments, the retaining strip comprises a pressure-sensitive adhesive. In some embodiments, the drawstring has two ends configured to be tied together to keep the second opening closed. In some embodiments, the at least one drawstring is unitary with the second end of the containment bag. In some embodiments, the at least one drawstring comprises at least one opening configured to be gripped by a user.

Some embodiments of the present containment assemblies comprise an improved containment assembly for a driver, the containment assembly including a coupler having a first end configured to be coupled to an intraosseous needle set and a second end configured to be coupled to a driver such that the driver can move the intraosseous needle set, the improvement of the containment assembly comprising a containment bag having: a first end having a first opening, the first end coupled to the coupler such that the coupler covers the first opening; and a second end having a second opening, where the containment bag is in a rolled configuration such that the second end can be rolled away from the first end to move the containment bag to an unrolled configuration, and where the containment bag is configured such that a driver can be coupled to the coupler and the second opening can be closed to seal the driver in the containment bag. In some embodiments, the containment bag is substantially sterile. In some embodiments, when a driver is coupled to the first end of the coupler, a user can move the containment bag into the unrolled configuration such that the driver is within an interior of the containment bag. In some embodiments, when the containment bag is in the unrolled configuration, the user can close the second opening by tying the second end.

Some embodiments of the present containment assemblies comprise an improved containment assembly for a driver, the containment assembly including a coupler having a first end configured to be coupled to an intraosseous needle set and a second end configured to be coupled to a driver such that the driver can move the intraosseous needle set, the improvement of the containment assembly comprising a substantially rigid casing comprising: a front portion having a first opening and a second opening, the front portion coupled to the coupler such that the coupler covers the first opening, and the second opening configured to receive a driver such that the driver can be coupled to the coupler; and a back portion movably coupled to the front portion, the back portion configured to cooperate with the front portion to accommodate a driver that is coupled to the coupler. In some embodiments, the casing is substantially sterile. In some embodiments, the first and second portions of the housing cooperate, when coupled together, to define a driver-shaped chamber. In some embodiments, the back portion is rotatably coupled to the front portion by a hinge pin. In some embodiments, the back portion is rotatably coupled to the front portion by a living hinge. In some embodiments, the back portion is detachably coupled to the front portion. In some embodiments, the front portion of the casing comprises a flexible membrane configured to permit a user to engage a trigger of a driver that is disposed in the casing.

Some embodiments of the present kits include a containment assembly having any of the characteristics previously described and an intraosseous needle set configured to be coupled to the coupler. In some embodiments, the kits comprise a driver configured to be coupled to the coupler. In some embodiments, the intraosseous needle set comprises at least one of a cannula and a stylet. In some embodiments, the kits comprise at least one sharps protector configured such that at least one of the cannula and the stylet can be disposed in the sharps protector to prevent exposure of a cutting surface.

Some embodiments of the present exemplary thods comprise inserting a driver into a containment bag of a containment assembly; coupling the driver to a coupler that is coupled to the containment bag, the containment bag having an opening and a rod positioned farther from the driver than is the opening; and rotating the rod toward the coupler to close the opening. In some embodiments, the methods further comprise coupling an intraosseous needle set to the coupler. In some embodiments, the exemplary methods comprise activating the driver to move the intraosseous needle set. In some embodiments, the driver is non-sterile when inserted into the containment bag.

Some embodiments of the present exemplary methods comprise inserting a driver into a containment bag of a containment assembly; coupling the driver to a coupler that is coupled to the containment bag, the containment bag having an opening; inserting a rod into a pocket of the containment bag that is spaced farther from the driver than is the opening; and rotating the rod toward the coupler to close the opening. In some embodiments, the methods further comprise coupling an intraosseous needle set to the coupler. In some embodiments, the exemplary methods comprise activating the driver to move the intraosseous needle set. In some embodiments, the driver is non-sterile when inserted into the containment bag.

Some embodiments of the present exemplary methods comprise inserting a driver into a containment bag of a containment assembly; coupling the driver to a coupler that is coupled to the containment bag, the containment bag having an opening; and moving a tab in a first direction to cause a first lockable track to engage a second lockable track to close the opening. In some embodiments, the exemplary methods further comprise coupling an intraosseous needle set to the coupler. In some embodiments, the exemplary methods comprise activating the driver to move the intraosseous needle set. In some embodiments, the driver is non-sterile when inserted into the containment bag.

Some embodiments of the present exemplary methods comprise inserting a driver into a containment bag of a containment assembly; coupling the driver to a coupler that is coupled to the containment bag, the containment bag having an opening; and pulling at least one drawstring to cinch the containment bag to close the opening. In some embodiments, the containment bag is prevented from moving to an unfolded configuration by at least one retaining strip coupled to the containment bag, and the exemplary method further comprises applying a sufficient force to decouple the at least one retaining strip from the containment bag to permit the containment bag to move into the unfolded configuration. In some embodiments, the exemplary methods comprise tying two ends of the at least one drawstring together to keep the containment bag closed. In some embodiments, the exemplary methods further comprise coupling an intraosseous needle set to the coupler. In some embodiments, the exemplary methods comprise activating the driver to move the intraosseous needle set. In some embodiments, the driver is non-sterile when inserted into the containment bag.

Some embodiments of the present exemplary methods comprise inserting a driver into a containment bag of a containment assembly; coupling the driver to a coupler that is coupled to the containment bag, the containment bag having an open end; unrolling the containment bag away from the coupler to move the containment bag to an unrolled configuration; and closing the open end of the containment bag. In some embodiments, the exemplary methods comprise tying the open end of the containment bag so as to seal the driver in an interior of the containment bag. In some embodiments, the exemplary methods further comprise coupling an intraosseous needle set to the coupler. In some embodiments, the exemplary methods comprise activating the driver to move the intraosseous needle set. In some embodiments, the driver is non-sterile when inserted into the containment bag.

Some embodiments of the present exemplary methods (e.g., of preventing desterilization of a target area) comprise inserting a driver into a front portion of a substantially rigid casing of a containment assembly; coupling the driver to a coupler that is coupled to the casing; and closing a back portion of the casing such that the back portion cooperates with the front portion of the casing to substantially enclose the driver. In some embodiments, the exemplary methods further comprise coupling an intraosseous needle set to the coupler. In some embodiments, the exemplary methods comprise activating the driver to move the intraosseous needle set. In some embodiments, the driver is non-sterile when inserted into the containment bag.

Any embodiment of any of the present containment assemblies, methods, and kits can consist of or consist essentially of - rather than comprise/include/contain/have - any of the described elements and/or features.

The feature or features of one embodiment may be applied to other embodiments, even though not described or illustrated, unless expressly prohibited by this disclosure or the nature of the embodiments. The invention is defined by the claims.

Details associated with the embodiments described above and others are presented below.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures illustrate the described elements using graphical symbols that will be understood by those of ordinary skill in the art. The embodiments of the present containment assemblies and their components shown in the figures are drawn to scale for at least the embodiments shown.
**FIG. 1A** depicts a perspective view of a prior art intraosseous device having a cannula and a stylet.
**FIG. IB** depicts a perspective view of another prior art cannula.
**FIGS. 1C** and **1D** depict perspective views of a prior art IO device having a stylet disposed in the cannula of **FIG. 1B****.**
**FIG. 2** depicts a cross-sectional side view of a prior art driver.
**FIG. 3** depicts a perspective view of the driver of **FIG. 2** with a prior art coupler assembly and a prior art IO device.
**FIG. 4** depicts the coupler assembly and IO device of **FIG. 3****.**
**FIG. 5** depicts portions of the driver of **FIG. 2** and the coupler assembly and a portion of the IO device of **FIG. 3****.**
**FIGS. 6A-6C** depict various views of the coupler assembly of **FIG. 3****.**
**FIGS. 7A-7C** depict various views of prior art kits.
**FIG. 8A** depicts an open configuration of a containment assembly having a containment bag and a rod.
**FIG. 8B** depicts a closed configuration of the containment assembly of **FIG. 8A****.**
**FIG. 9A** depicts an open configuration of a containment assembly having a containment bag and a tab.
**FIG. 9B** depicts a closed configuration of the containment assembly of **FIG. 9A****.**
**FIG. 10A** depicts an open configuration of a containment assembly having a containment bag and at least one (e.g., two) drawstrings.
**FIG. 10B** depicts a partially closed configuration (e.g., partially cinched) of the containment assembly of **FIG. 10A****.**
**FIG. 11A** depicts an open configuration (e.g., a rolled configuration) of a containment assembly having a containment bag with a rollable and/or unreliable second end.
**FIG. 11B** depicts a partially closed configuration (e.g., partially unrolled) of the containment assembly of **FIG. 11A****.**
**FIG. 11C** depicts a closed configuration (e.g., an unrolled configuration) of the containment assembly of **FIG. 11A****.**
**FIG. 12A** depicts an open configuration of a containment assembly, in accordance with the present claimed invention, having a substantially rigid casing with a movable back portion.
**FIG. 12B** depicts a closed configuration of the containment assembly of **FIG. 12A****.**

### DESCRIPTION OF ILLUSTRATIVE EMBODIMENTS

The term "coupled" is defined as connected, although not necessarily directly, and not necessarily mechanically. Two items are "couplable" if they can be coupled to each other. Unless the context explicitly requires otherwise, items that are couplable are also decouplable, and vice-versa. One non-limiting way in which a first structure is couplable to a second structure is for the first structure to be configured to be coupled (or configured to be couplable) to the second structure. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the terms "substantially," "approximately," and "about" may be substituted with "within [a percentage] of' what is specified, where the percentage includes 0.1, 1, 5, and 10 percent.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a containment assembly or a kit, or components of a containment assembly or a kit that "comprises," "has," "includes" or "contains" one or more elements or features possesses those one or more elements or features, but is not limited to possessing only those elements or features. Likewise, a method that "comprises," "has," "includes" or "contains" one or more steps possesses those one or more steps, but is not limited to possessing only those one or more steps. Additionally, terms such as "first" and "second" are used only to differentiate structures or features, and not to limit the different structures or features to a particular order.

Further, a containment assembly and kits and methods comprising containment assemblies configured in a certain way are configured in at least that way, but can also be configured in other ways than those specifically described. Embodiments of the present powered drivers may be used to insert an IO device into a selected target area or target site in ten seconds or less. However, various teachings of the present disclosure are not limited to use with powered drivers. Manual drivers and spring powered drivers may also be used with IO devices and embodiments of the present containment assemblies incorporating teachings of the present disclosure.

Various types of coupler assemblies incorporating teachings of the present disclosure may be satisfactorily used to releasably engage one end of a shaft extending from a driver with one end of an intraosseous device. For some embodiments, the powered driver may include a driveshaft having one end with a generally hexagonal cross section operable to be releasably engaged with a latch mechanism disposed in one end of a coupler assembly. For some embodiments, a coupler assembly incorporating teachings of the present disclosure may be referred to as a "hands free" coupler, a quick disconnect or quick release coupler and/or port assembly.

Examples of manual drivers are shown in co-pending patent application serial No. 11/042,912 entitled Manual Intraosseous Device filed January 25, 2005 (published as US 2005/0165404). The term "fluid" may be used in this application to include liquids such as, but not limited to, blood, water, saline solutions, IV solutions, plasma, or any mixture of liquids, particulate matter, dissolved medication, and/or drugs associated with biopsy or aspiration of bone marrow or communication of fluids with bone marrow or other target sites. The term "fluid" may also be used in this patent application to include any body fluids and/or liquids containing particulate matter such as bone marrow and/or cells which may be withdrawn from a target area.

The terms "harvest" and "harvesting" may be used in this application to include bone and/or bone marrow biopsy and bone marrow aspiration. Bone and/or bone marrow biopsy (sometimes referred to as "needle biopsy") may be generally described as removing a relatively small piece or specimen of bone and/or bone marrow from a selected target area for biopsy purposes. Bone marrow aspiration (sometimes referred to as "bone marrow sampling") may be generally described as removing larger quantities of bone marrow from a selected target area. Relatively large quantities of bone marrow may be used for diagnostic, transplantation, and/or research purposes. For example some stem cell research techniques may require relatively large quantities of bone marrow.

The term "insertion site" may be used in this application to describe a location on a bone at which an intraosseous device may be inserted or drilled into the bone and associated bone marrow. Insertion sites are generally covered by skin and soft tissue. The term "target area" refers to any location on or within biological material, such as the biological material of a living human being.

The term "intraosseous (IO) device" may be used in this application to include, but is not limited to, any hollow needle, hollow drill bit, penetrator assembly, bone penetrator, catheter, cannula, trocar, stylet, inner penetrator, outer penetrator, IO needle, biopsy needle, aspiration needle, IO needle set, biopsy needle set or aspiration needle set operable to provide access to an intraosseous space or interior portions of a bone. Such IO devices may be formed, at least in part, from metal alloys such as 304 stainless steel and other biocompatible materials associated with needles and similar medical devices.

Embodiments of the present containment assemblies can be included in medical procedure trays such as those disclosed in International Patent Application No. PCT/US2007/078207 (published as WO 2008/033874).

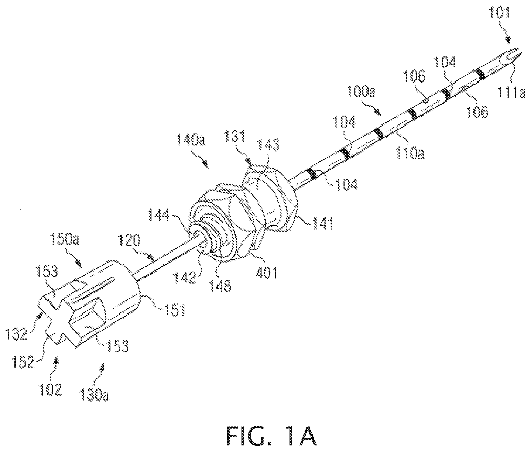

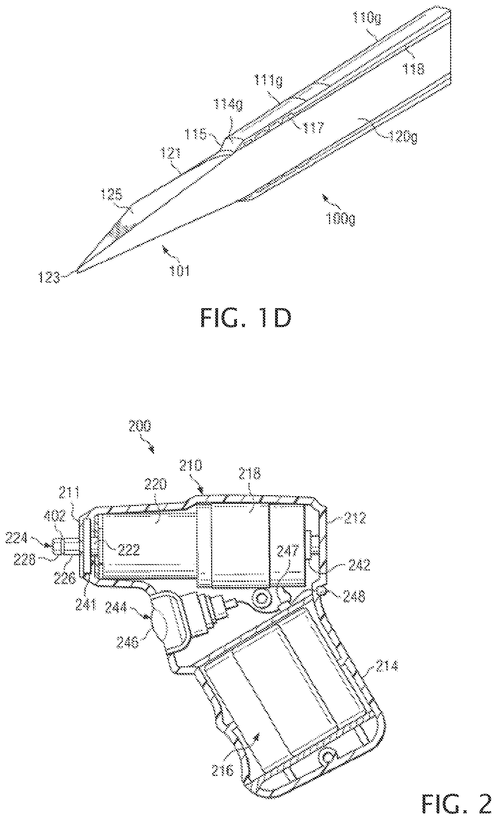

The devices and components shown in FIGS. 1A to 7C are prior art devices and components, and the following description of them is provided to give the reader context for the types of devices and components that can be used consistently with embodiments of the present containment assemblies, kits, and methods.

Referring now to the drawings, and more particularly to FIG. 1A, shown therein and designated by the reference numeral 100 is one embodiment of the present intraosseous (IO) needle sets or aspiration needle sets. Aspiration needle set 100 comprises a hollow outer penetrator or cannula 110a, a corresponding inner penetrator or stylet (or trocar) 120, and a hub assembly 130a. In the embodiment shown, first end 111a of cannula 110a and first end 121 of stylet 120 are operable or configured to penetrate a bone and associated bone marrow. Various features of first end 111a of cannula 110a and first end 121 of stylet 120 are shown in more detail in. First end 101 of IO needle set 100 corresponds generally with first end 111a of cannula 110a and first end 121 of stylet 120.

In the embodiment shown, cannula 110a includes a plurality of markings 104 disposed on exterior portions of the cannula. Markings 104 may be referred to as "positioning marks" or "depth indicators," and may be used to indicate the depth of penetration of needle set 100 into a bone and associated bone marrow. In some embodiments, cannula 110a may have a length of approximately sixty (60) millimeters and/or a nominal outside diameter of approximately 0.432 mm (0.017 inches) (e.g., corresponding generally to the dimensions of a sixteen (16) gauge needle). Cannula 110a and/or stylet 120 may be formed from stainless steel or other suitable biocompatible materials. In some embodiments, markings 104 are spaced at one (1) centimeter intervals on exterior portions of cannula 110a. In some embodiments, one or more side ports 106 may be formed in exterior portions of cannula 110a spaced from first end 111a.

Hub assembly 130a may be configured and/or used to releasably dispose stylet 120 within the longitudinal bore or lumen of cannula 110a. In the embodiment shown, hub assembly 130a includes a first hub 140a and a second hub 150a. A second end of cannula 110a, opposite from first end 111a, may be securely engaged with hub 140a. The second end of stylet 120, opposite from first end 121, may be securely engaged with the first end of hub 150a. As shown in FIG. 1A, cannula 110a may extend longitudinally from first end 141 of hub 140a. Stylet 120 may also extend from the first end of hub 150a. The second end of hub 140a may include a standard Luer lock fitting which may be releasably engaged with a corresponding Luer lock fitting disposed within the first end of second hub 150a. The Luer lock fitting disposed on the second end of hub 140a may be in fluid communication with the bore or passage in cannula 110a, and may be operable to be releasably engaged with a standard syringe type fitting and/or a standard intravenous (IV) connection. In the embodiment shown, hub 150a includes second end 152 that generally corresponds with second end 132 of hub assembly 130a and second end 102 of IO needle set 100. Hub 140a may include first end 141 which may generally correspond with first end 131 of hub assembly 130a. Cannula 110a may extend longitudinally from first end 141 of hub 140a and first end 131 of hub assembly 130.

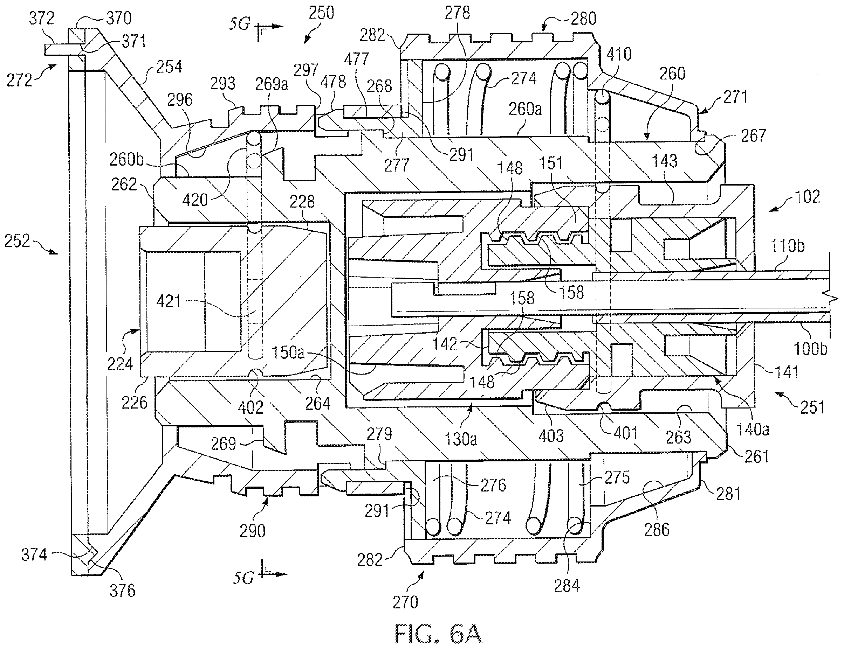

In the embodiment shown, the second end of a hub assembly may be operable to be disposed within a receptacle formed in a coupler assembly, as described in more detail below. One feature of the present disclosure may include forming a hub assembly which may be releasably engaged within a first receptacle disposed in a first end of a coupler assembly (e.g., receptacle 263 proximate first end 261 of elongated core 260 as shown in FIGS. 6A-6B). The dimensions and configuration of receptacle 263 may be selected to prevent rotation of hub 150a relative to hub 140a if hub assembly 130a is disposed in receptacle 263 (e.g., while inserting (rotating) an IO device into a bone and associated bone marrow). A powered driver may be releasably engaged with a second receptacle disposed in a second end of the coupler assembly (e.g., receptacle 264 proximate second end 262 of elongated core 260 as shown in FIGS. 6A-6B).

In the embodiment shown, intraosseous device or aspiration needle set 100a includes first end 151 of hub 150a spaced from second end 142 of hub 140a. Portions of stylet 120 extending from first end 151 of hub 150a are shown slidably disposed within lumen or longitudinal bore 118 of cannula 110a. Hub assembly 130a may include first end 131 which may correspond generally with first end 141 of hub 140a. Hub assembly 130a may also include second end 132 which may correspond generally with second end 152 of hub 150a and second end 102 of hub assembly 130a, as shown. Cannula 110a may be attached to and extend from first end 141 of hub 140a. Second end 142 of hub 140a may include one-half a typical Luer lock connection or fitting operable to be releasably engaged with corresponding portions of a Luer lock connection or fitting disposed in first end 151 of second hub 150a. For embodiments such as the one shown in FIG. 1A, first end 131 of hub assembly 130a may correspond with first end 141 of first hub 140a. Second end 152 of second hub 150a may correspond with second end 132 of hub assembly 130a and second end 102 of aspiration needle set 100a.

At least one portion of hub assembly 130a may have a generally hexagonal cross section operable to be received within the generally hexagonal cross section of receptacle 263 disposed proximate first end 251 of coupler assembly 250, as shown in FIGS. 6A-6B. For some embodiments, portions of first hub 140a disposed adjacent to reduced outside diameter portion 143 may have generally hexagonal cross sections, as shown in FIG. 1A. In other embodiments, various cross sections other than hexagonal may be satisfactorily used to releasably engage a powered driver with one end of a coupler assembly and an intraosseous device with an opposite end of the coupler assembly. Aspiration needle sets may include a stylet, stylet or penetrator in combination with an associated cannula, catheter or outer penetrator. However, biopsy needles formed in accordance with teachings of the present disclosure may or may not include a stylet, stylet or inner penetrator.

Hub 140a may include second end 142 with opening 144 formed therein. A passageway may extend from second end 142 towards first end 141 of hub 140a, as illustrated in FIGS. 6A-6B. A passageway may be operable to communicate fluids with lumen 118 of cannula 100a. Second end 142 of hub 140 may include various features of a conventional Luer lock connection or fitting, including threads 148, and corresponding threads 158 may be formed within first end 151 of hub 150a, as shown in FIGS. 6A-6B.

For some applications hub 140a and hub 150a may, for example, be formed using injection molding techniques. For such embodiments hub 140a may include reduced outside diameter portion 143 disposed between first end 141 and second end 142. In a similar manner a plurality of void spaces or cutouts 153 may be formed in hub 150a adjacent to and extending from second end 152 in the direction of first end 151. The configuration and dimensions of reduced diameter portion 143 and/or cutouts 153 may be varied to optimize associated injection molding techniques and at the same time provide required configurations, dimensions and material strength to allow associated hub assembly 130a to function as described in this disclosure.

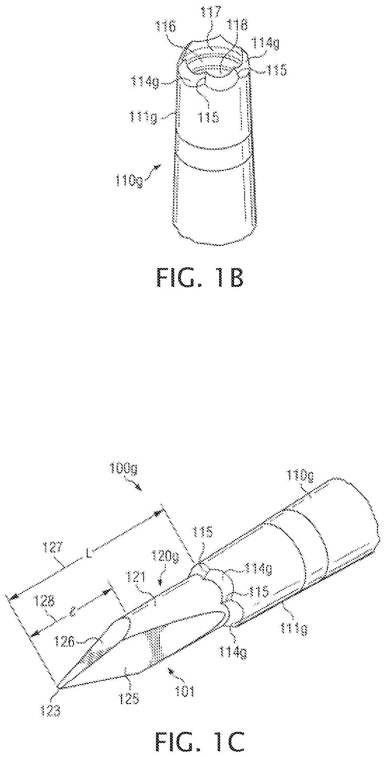

In some embodiments, tip 123 of stylet 120 may be disposed relatively close to a tip of cannula 110a. For some applications, first end 121 of stylet 120 and first end 111a of cannula 110a may be ground at the same time to form adjacent cutting surfaces. Grinding ends 111a and 121 at the same time may result in forming a single cutting unit to form generally matching cutting edges. Other types of cutting surfaces formed in accordance with teachings of the present disclosure may be discussed later (e.g., as described with reference to FIGS. 1B-1D).

FIGS. 1B-1D show a second example of cutting surfaces and tips which may be formed adjacent to the ends of a cannula and/or an associated stylet in the present embodiments. In the embodiment shown, outer penetrator or cannula 110g may include first end 111g having a plurality of cutting surfaces 114g formed adjacent to opening 116 in first end 111g. Opening 116 may communicate with and form a portion of an associated longitudinal bore or lumen 118. For some applications cutting surfaces 114g may be formed using electrical discharge machining (EDM) techniques or otherwise, as described in WO 2008/033874. In the embodiment shown, first end 111g has a generally tapered configuration or reduced outside diameter as compared with other portions of cannula 110g In other embodiments, first end 111g has an outside diameter that is equal to the outside diameter of other portions of cannula 110g (e.g., cannula 110g can have a constant outside diameter along the entire length of the cannula). Cutting surfaces 114g may, for example, be formed using machine grinding techniques. In some embodiments, such as the one shown, end 111g of cannula 110g may include six ground cutting surfaces 114g with respective crowns 115 therebetween. Forming a biopsy needle set and/or biopsy needle with tapered end 111g and a plurality of cutting surfaces 114g and crowns 115 may provide improved drilling performance (e.g., relative to others configurations) when the resulting biopsy needle set and/or biopsy needle is used with a powered driver in accordance with teachings of the present disclosure. For some applications, a helical groove 117 may be formed within longitudinal bore 118 proximate opening 116. Helical groove 117 may assist with retaining a biopsy specimen or a bone marrow specimen within longitudinal bore 118. For example, a single thread may be disposed within the longitudinal bore or lumen of the cannula such that the helical groove 117 is defined between turns of the thread. Various techniques and procedures may be satisfactorily used to place the single thread or otherwise form the helical groove, as described WO 2008/033874.

As shown in FIG. 1C, a biopsy needle set 100g may include cannula or outer penetrator 110g with stylet or inner penetrator 120g slidably disposed therein. The proximal ends of cannula 110g and stylet 120g may be similar to those of cannula 110a and stylet 120 depicted in FIG. 1A (e.g., may include hubs 140a and 150a, respectively). For some applications first end 101 of biopsy needle set 100g may minimize damage to skin and soft body tissue at an insertion site. For some applications inner penetrator or stylet 120g may include first end 121 having a plurality of cutting surfaces 125 and 126 formed on exterior portions thereof extending from associated tip 123 towards second end of stylet or inner penetrator 120g. For some applications one or more cutting surfaces 125 may be formed having length 127 extending from tip 123 to associated cutting surfaces 114g in associated cannula 110g. One or more cutting surfaces 126 may be formed adjacent to each cutting surface 125 with second length 128. First length 127 may be greater than second length 128. As shown, lengths 127 and 128 are measured parallel to the central longitudinal axis of stylet 120g. The ratio of first length 127 and second length 128 may be varied in accordance with teachings of the present disclosure to provide optimum performance for penetrating a selected bone and associated bone marrow. Additional details of some embodiments of first end 101 are described in WO 2008/033874.

FIG. 2 depicts a cross-sectional view of one embodiment of a driver that can be used with embodiments of the present containment assemblies and kits. In the embodiment shown, powered driver 200 may be used to insert an intraosseous devices into a bone and associated bone marrow. Powered driver 200 may include housing 210 having a general configuration similar to a small pistol defined in part by handle 214. Various components associated with powered driver 200 may be disposed within housing 210 (e.g., handle 214). For example a power source such as battery pack 216 may be disposed within handle 214. Housing 210 may be formed from relatively strong, heavy duty polymeric materials such as polycarbonate or other satisfactory materials. For some applications housing 210 may be formed in two halves (not expressly shown) which may be joined together with a fluid tight seal to protect various components of powered driver 200 disposed therein.

Motor 218 and gear assembly 220 may be disposed within portions of housing 210 adjacent to handle 214. Motor 218 and gear assembly 220 may be generally aligned with each other. Motor 218 may be rotatably engaged with one end of gear assembly 220. Drive shaft 222 may be rotatably engaged with and extend from another end of gear assembly 220 opposite from motor 218. For some applications both motor 218 and gear assembly 220 may have generally cylindrical configurations. Distal end or first end 211 of housing 210 may include an opening with portions of drive shaft 222 extending through the opening, as shown. For some applications, end 224 or the portion of drive shaft 222 extending from first end 211 of housing 210 may have a generally hexagonal cross section with surfaces 226 disposed thereon. Receptacle 263 disposed in second end 252 of coupler assembly 250 may have a matching generally hexagonal cross section, as shown in FIGS. 6A-6C.

Surfaces 226 may extend generally parallel with each other and parallel with respect to a longitudinal axis or rotational axis of drive shaft 222. One or more tapered surfaces 228 may also be formed on end 224 to assist with releasably engaging powered driver 200 with coupler assembly 250. Embodiments of powered driver 200 include speed reduction ratios, for example, of between 60:1 and 80:1, resulting in drive shaft RPMs that are reduced relative to motor RPMs. Coupler assemblies having corresponding openings or receptacles may be releasably engaged with end 224 extending from first end 211 of powered driver 200. For example, end 224 extending from first end 211 of housing 210 may be releasably engaged with receptacle 264 disposed proximate second end 252 of coupler assembly 250, as shown in FIGS. 6A-6B.

For some applications thrust bearing 241 may be disposed between first end or distal end 211 of housing 210 and adjacent portions of gear assembly 220. Thrust bearing 242 may be disposed between second end or proximal end 212 of housing 210 and adjacent portions of motor 218. Thrust bearings 241 and 242 may limit longitudinal movement of motor 218, gear assembly 220 and drive shaft 222 within associated portions of housing 210. Trigger assembly 244 may also be disposed within housing 210 proximate handle 214. Trigger assembly 244 may include trigger or contact switch 246. Motor 218 may be energized and deenergized by alternately depressing and releasing trigger 246. Electrical circuit board 247 may also be disposed within housing 210. Electrical circuit board 247 may be electrically coupled with trigger assembly 244, motor 218, power supply 216 and indicator light 248. For some applications indicator light 248 may be a light emitting diode (LED) or a small more conventional light bulb. For some applications indicator light 248 may be activated when ninety percent (90%) of electrical storage capacity of battery pack 216 has been used. The configuration and dimensions of an intraosseous device formed in accordance with teachings of the present disclosure may vary depending upon respective intended applications for each intraosseous device. For example the length of a biopsy needle formed in accordance with teachings of the present disclosure may vary from approximately five (5) millimeters to thirty (30) millimeters.

Coupler assemblies incorporating teachings of the present disclosure may function as "quick release mechanisms" operable to engage and disengage an IO device from a powered driver (e.g., a driver disposed within a flexible containment bag or sterile sleeve). Such coupler assemblies may allow rotation of an IO device (e.g., biopsy needle or needle set) without damage to the flexible containment bag or sterile sleeve. One end of the coupler assembly may be operable to form a fluid seal or fluid barrier with adjacent portions of the containment bag or sterile sleeve. A coupler assembly incorporating teachings of the present disclosure may also be described as a port assembly attached to a containment bag. Such port assemblies may allow easy engagement or disengagement of a powered driver from an IO device and at the same time allow the powered driver to "power in and power out" an IO device from an insertion site.

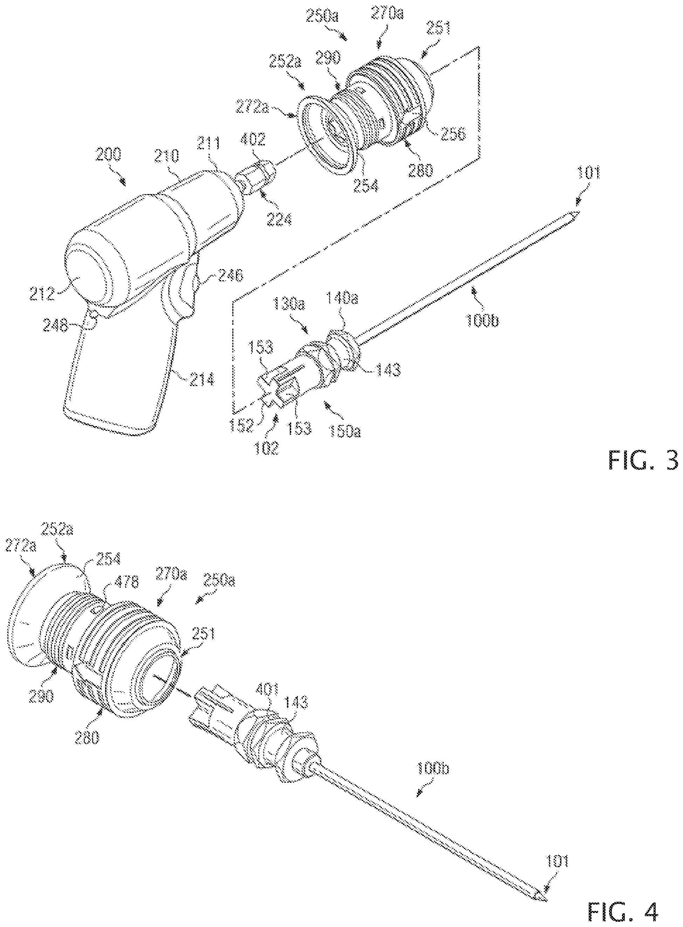

FIGS. 3-6C depict an example of a coupler assembly 250 suitable for some embodiments of the present assemblies and kits. FIGS. 3-5 are perspective views showing various views of powered driver 200, coupler assembly 250a, and intraosseous device 100b that is substantially similar to device 100a with the exception that device 100b does not include markings 104. Coupler assembly 250a includes a first end 251 operable to be releasably engaged with one end of an intraosseous device such as, but not limited to, second end 102 of biopsy needle set 100b. Coupler assembly 250a also includes a second end 252 operable to be releasably engaged with a portion of a drive shaft extending from a powered driver, such as, but not limited to, end 224 of drive shaft 222 extending from first end 211 of housing 210 of powered driver 200. Though not depicted here, second end 252 of coupler assembly 250 may be securely engaged with an opening in a containment bag or sterile sleeve, as described in WO 2008/033874.

Coupler assemblies incorporating various teachings of the present disclosure may be placed in a medical procedure tray or kit with one end down and an opposite end looking up to allow "hands free" releasable engagement with a powered driver or a manual driver. For example, coupler assembly 250a may be disposed in medical procedure tray with first end 251 facing downward and second end 252 facing up such that end 224 of drive shaft 222 (of driver 200) may be inserted into and releasably engaged with second end 252 of coupler assembly 250 without requiring an operator or user to physically contact or manipulate any portion of coupler assembly 250a. As described below, coupler 250a may include a "hands free" latching mechanism.

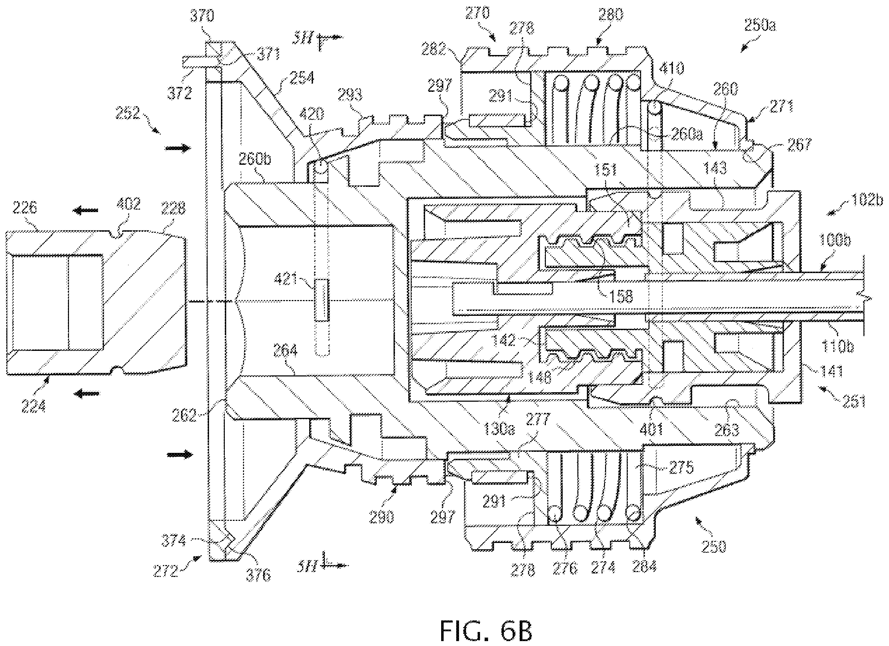

In the embodiment shown, coupler assembly 250a may include elongated core 260 with housing assembly 270 slidably disposed on exterior portions of elongated core 260. Housing assembly 270/270a may include first end 271 and second end 272 which may be generally aligned with respective first end 261 and respective second end 262 of elongated core 260. For some applications, elongated core 260 may have a generally cylindrical configuration defined in first exterior portion 260a and second exterior portion 260b with various shoulders and/or recesses formed thereon. For some embodiments first exterior portion 260a may have a larger diameter than second exterior portion 260b. Housing assembly 270 may be described as having a generally hollow, cylindrical configuration defined in part by first housing segment 280 and second housing segment 290. The first end of housing segment 280 may generally correspond with first end 271 of housing assembly 270. The second end of second housing segment 290 may generally correspond with second end 272 of housing assembly 270. First end 291 of second housing segment 290 may be described as having a generally cylindrical configuration with an outside diameter smaller than the adjacent inside diameter of second end 282 of first housing segment 280. Second housing segment 290 may slide longitudinally from a first position (FIG. 6A) to a second position (FIG. 6B) within second end 282 of first housing segment 280 to release one end of a drive shaft engaged with second end 252 of coupler assembly 250.

A biasing mechanism such as coiled spring 274 may be disposed around exterior portion 260a of generally elongated core 260. First end 275 of coiled spring 274 may contact annular shoulder 284 formed on interior portions of first housing segment 280. Second end 276 of coiled spring 274 may contact annular shoulder 278 disposed proximate first end 291 of second housing segment 290. Coil spring 274, annular shoulder 284 and annular shoulder 278 may cooperate with each other to generally maintain first housing segment 280 and second housing segment 290 in a first extended position relative to each other. Other biasing mechanisms such as, but not limited to, leaf springs and bellows (not expressly shown) may also be disposed between annular shoulder 284 and annular shoulder 278. Annular shoulder 278, associated with second end 276 of coiled spring 274, may extend radially outward from generally cylindrical ring 277. Generally cylindrical ring 277 may be slidably and rotatably disposed on exterior portion 260a of elongated core 260. Annular shoulder 279 may be disposed on interior portions of generally cylindrical ring 277 and may extend radially inward toward adjacent portions of elongated core 260. Annular shoulder 268 may be formed on exterior portion 260a of elongated core 260 intermediate first end 261 and second end 262. The configuration and dimensions of annular shoulder 268 and annular shoulder 279 are selected to be compatible with each other such that engagement between annular shoulder 279 of generally cylindrical ring 277 with annular shoulder 268 of elongated core 260 may limit movement of second housing segment 290 longitudinally in the direction of second end 262 of elongated core 260.

For some applications a plurality of flexible collets or fingers 477 may extend from generally cylindrical ring 277 opposite from annular shoulder 278. Respective collet heads 478 may be formed on the end of each collet 477 opposite from annular shoulder 278. The dimensions and configuration of collet heads 478 may be selected to be received within respective slots or openings 297 formed in second housing 290. During manufacture of coupler assembly 250a, each collet head 478 may be disposed within respective slot or opening 297 to securely engage generally cylindrical ring 277 and annular shoulder 278 proximate first end 291 of second housing segment 290. As a result, second housing segment 290 and annular shoulder 278 may generally move as a single unit relative to elongated core 260 and first housing segment 280. During disengagement of an intraosseous device from first end 251 of coupler assembly 250a, first housing segment 280 may move or slide longitudinally toward second housing segment 290. In a similar manner, second housing segment 290 may move or slide longitudinally toward first housing segment 280 during disengagement of a powered driver from second end 252 of coupler assembly 250a.

Annular shoulder 267 may be formed on exterior portions of elongated core 260 proximate first end 261. Annular shoulder 267 may engage portions of first end 271 of housing 270 to limit longitudinal movement of first housing segment 280 during longitudinal movement of second housing segment 290 towards first end 261 of elongated core 260 during disengagement of a powered driver from second end 252 of coupler assembly 250a. As previously noted, annular shoulder 268 may be formed on exterior portions of elongated core 260 between first end 261 and second end 262. Engagement between annular shoulder 268 and annular shoulder 279 of generally cylindrical ring 277 may limit movement of second housing segment 290 toward second end 262 of elongated core 260. Contact between spring 274 and annular shoulder 278 and annular shoulder 284 of first housing segment 280 may limit the longitudinal movement of first housing segment 280 in the direction of second end 262 of elongated core 260 during disengagement of an intraosseous device from first end 251 of coupler assembly 250a.

Generally cylindrical ring 277 and attached annular shoulder 279 may slide longitudinally on exterior portions of annular core 260 between annual shoulder 268 and annular shoulder 267. First housing segment 280 may move longitudinally toward second end 262 of elongated core 260 to release one end of intraosseous device from engagement with first end 251 of coupler assembly 250a. In a similar manner, second housing segment 290 may move longitudinally toward first end 261 of elongated core 260 to release one end of a drive shaft extending from a powered driver engaged with second end 252 of coupler assembly 250a. A wide variety of latches and latch mechanisms may be satisfactorily used to releasably engage one end of an intraosseous device within a first end of a coupler assembly incorporating teachings of the present disclosure. In a similar manner, a wide variety of latches and latch mechanisms may be satisfactorily used to releasably engage one end of a drive shaft extending from a powered driver or manual driver within a second end of the coupler assembly incorporating teachings of the present disclosure.

For embodiments represented by coupler assembly 250a, first latch 410 may be disposed on exterior portions of elongated core 260 proximate receptacle 263 adjacent to first end 261 to releasably engage one end of an IO device such as second end 102 of biopsy needle set 100b within receptacle 263 of coupler assembly 250a. Second latch mechanism 420 may be disposed on exterior portions of elongated core 260 proximate receptacle 264 adjacent to second end 262 to releasably engage one end of a drive shaft with second end 252 of coupler assembly 250a. Second latch 420 may be used to releasably engage one portion of a drive shaft such as end 224 of drive shaft 222 extending from powered driver 200 within second end 252 of coupler assembly 250a. Latch 410 may releasably engage an intraosseous device with first end 251 of coupler assembly 250a and substantially the same latch 420 may releasably engage a powered driver with second end 252 of coupler assembly 250a.

For some applications, latches 410 and 420 may have similar configurations such as a general "omega" shape (e.g., latch 420). However, latch 410 may have larger dimensions corresponding generally with exterior portion 260a of elongated core 260. Latch 420 may have smaller dimensions corresponding generally with exterior portion 260b of elongated core 260. Various features of the present disclosure may be described with respect to latch mechanism 420 along with adjacent portions of second housing segment 290 and exterior portion 260b of elongated core 260. Respective detents 421 and 422 may be formed on opposite ends of generally omega shaped latch 420. In a similar manner, respective detents (not expressly shown) may be formed on the ends of generally omega shaped latch 410. The configuration and dimensions of detents 421 and 422 may be compatible with placing each detent 421 and 422 in a respective slot or opening extending between exterior portion 260b of elongated core 260 to interior portions of receptacle 264 disposed proximate second end 252 of coupler assembly 250a. Latch 420 may have a first position in which portions of detents 421 and 422 may extend through the respective slots. The dimensions and configuration of detent 421 and 422 may be operable to be securely engaged with annular groove 402 formed in end 224 of powered driver 200. In a similar manner, respective detents on associated latch 410 may be releasably engaged with annular groove 401 disposed in second end 102 of biopsy needle 100b. For some applications, a plurality of tapered surfaces 403 may be formed on exterior portions of hub 140a proximate first end 142 to radially expand detent mechanisms associated with omega shaped latch 410 radially outward while inserting second end 102 of biopsy needle 100b into first end 251 of coupler assembly 250a. The detent mechanism may "snap" into annular groove 401 when aligned therewith. In a similar manner, a plurality of tapered surfaces 228 may be formed on exterior portions of end 224 of drive shaft 222 extending from powered driver 200 to radially expand detent mechanisms 421 and 422 radially outward during the insertion of end 224 of powered driver 200 into second end 252 of coupler assembly 250a. Detent mechanisms 421 and 422 will "snap" into annular groove 402 when aligned therewith.

Engagement between detent mechanisms associated with latch 410 with annular groove 401 of hub assembly 130a will generally retain second end 102 of biopsy needle 100b securely engaged with first end 251 of coupler assembly 250a. This engagement may allow powered driver 200 to rotate or spin cannula or biopsy needle 110b while withdrawing cannula or biopsy needle 110b from an insertion site. In a similar manner, engagement between detent mechanisms 421 and 422 of omega shaped latch 420 and annular groove 402 of end 224 of powered driver 200 will generally retain second end 252 of coupler assembly 250a engaged with powered driver 100 during withdrawal of cannula 110b from an insertion site.

Biopsy needle set 100b may be released from first end 251 of coupler assembly 250a by sliding first housing segment 280 longitudinally toward second end 262 of elongated core 260. Such movement of first housing segment 280 will result in interior tapered surface 286 contacting exterior portions of omega shaped latch 410 and compressing omega shaped latch 410 to radially expand associated detent mechanisms (not expressly shown) from engagement with annular groove 401 of hub assembly 130a. As a result, biopsy needle set 100b may be easily withdrawn from first end 251 of coupler assembly 250a. In a similar manner, longitudinal movement of second housing segment 290 toward first end 251 of coupler assembly 250a will result in interior tapered surface 296 contacting exterior portions of omega shaped latch 420 to compress generally omega shaped latch 420 and withdraw or retract detent mechanisms 421 and 422 from engagement with annular groove 402 of end 224. As a result, powered driver 200 and second end 222 of coupler assembly 250a may be easily disconnected from each other.

Flange 254 may be generally described as having an enlarged funnel shaped or bell shaped configuration. The dimensions and configuration of flange 254 may be selected to be compatible with end 211 of powered driver 200. As previously noted, coupler assembly 250a may be securely engaged with an opening formed in a containment bag or sterile sleeve in accordance with teachings of the present disclosure. For embodiments such as the one shown, end 272 of housing 270 of coupler assembly 250a may include annular ring 370 operable to be securely engaged with adjacent portions of flange 254. The outside diameter of annular ring 370 may generally correspond with the outside diameter of adjacent portions of flange 254. The inside diameter of annular ring 370 may also generally correspond with the inside diameter of adjacent portions of flange 254. For some embodiments a plurality of posts 372 and generally V shaped grooves 374 may be alternatingly disposed on the extreme end of flange 254. Annular ring 370 may include a plurality of holes 371 sized to received respective posts 372 therein. Annular ring 370 may also include a plurality of generally V shaped projections 376 sized to be received within respective generally V shaped grooves 374 formed in adjacent portions of flange 254. For embodiments such as the one shown, portions of a containment bag (e.g., around an opening) may be disposed between annular ring 370 and adjacent portions of flange 254. For example, post 372 may be inserted through a corresponding hole in a containment bag adjacent to the perimeter of an opening in the containment bag. Holes 371 in annular ring 370 may be aligned with respective posts 372. Other portions of a containment bag (e.g., adjacent to an opening) may be trapped between respective V shaped projections 376 and V shaped grooves 374. Various welding techniques including, but not limited to, laser welding may be applied to posts 372 to bond annular ring 370 with adjacent portions of flange 354. As a result, a perimeter of a containment bag around an opening in the containment bag may be securely engaged with second end 252 of coupler assembly 250a.

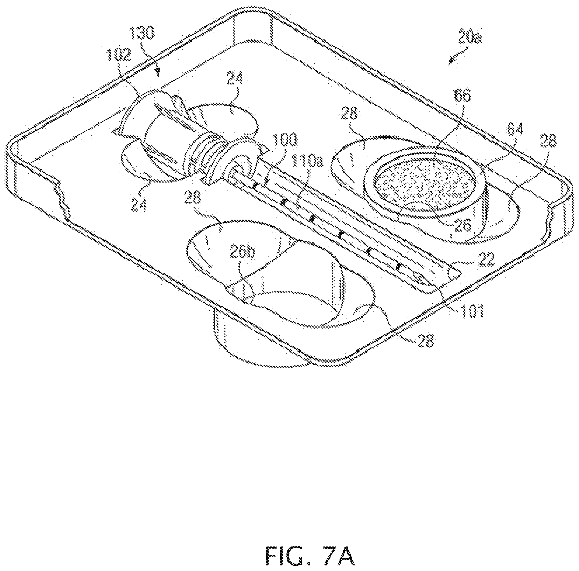

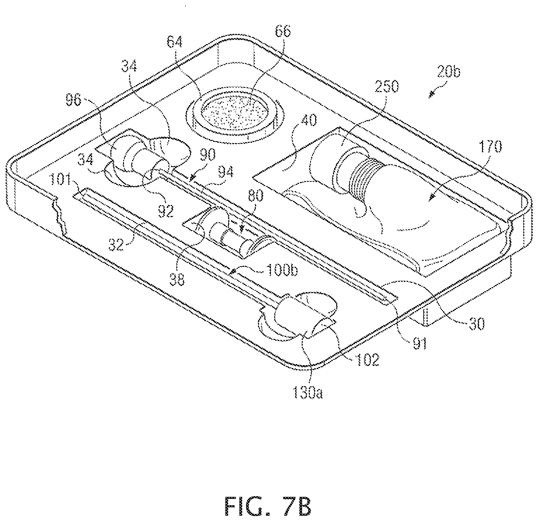

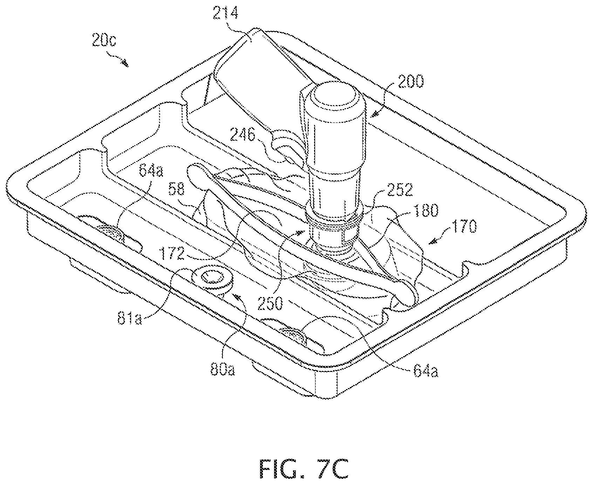

FIGS. 7A-7C show some examples of medical procedure trays and/or kits which may contain one or more intraosseous devices and/or other components incorporating teachings of the present disclosure. For example, medical procedure tray 20a as shown in FIG. 7A may include intraosseous needle set or aspiration needle set 100 incorporating various teachings of the present disclosure. Medical procedure tray 20b as shown in FIG. 7B may include intraosseous needle set or biopsy needle set 100b, ejector 90, funnel 80 and/or containment bag or sterile sleeve 170. Medical procedure tray 20c as shown in FIG. 7C may also include various IO devices and other components incorporating teachings of the present disclosure including, but not limited to, biopsy needle set 100b, coupler assembly 250, containment bag 170, ejector 90 and/or funnel 80a.

Medical procedure trays and/or kits formed in accordance with teachings of the present disclosure may provide a support or base for various components such as a coupler assembly, funnel, and/or sharps protector to allow an operator or user to perform various functions without requiring that the operator or user hold or manipulate the respective component. For example, medical procedure tray 20c as shown in FIG. 7C may position and support coupler assembly 250 such that one end of a powered driver may be inserted (pushed) into releasable engagement with second end 252 of coupler assembly 250. The powered driver may then be used to withdraw coupler assembly 250 from medical procedure tray 20c without requiring an operator or user to directly hold or manipulate coupler assembly 250.

Medical procedure trays 20a, 20b and/or 20c may also contain a wide variety of other components including, but not limited to, one or more sharps protectors 64 as shown in FIGS. 7A and 7B. Sharps protectors 64 may include hard foam or claylike material 66 disposed therein. Intraosseous devices such as aspiration needle sets and biopsy needle sets typically have respective sharp tips and/or cutting surfaces operable to penetrate skin, soft tissue and bone. The sharp tips and/or cutting surfaces of such intraosseous devices may be inserted into hard foam or claylike material 66 after completion of a medical procedure using the respective intraosseous device.

FIG. 7C shows one procedure for placing a powered driver within a containment bag incorporating teachings of the present disclosure. Containment bag 170 may be formed from generally flexible, fluid impervious material which may also be sterilized using conventional sterilization techniques. Containment bag 170 may be used to prevent a non-sterile powered driver from contaminating a sterile intraosseous device and/or an injection site, particularly during a bone marrow biopsy procedure or a bone marrow aspiration procedure. Containment bag 170 may be operable to form a fluid barrier with adjacent portions of housing assembly 270. At the same time, coupler assembly 250 may allow powered driver to rotate an intraosseous device releasably engaged with first end 251 of coupler assembly 250 without damage to containment bag 170.

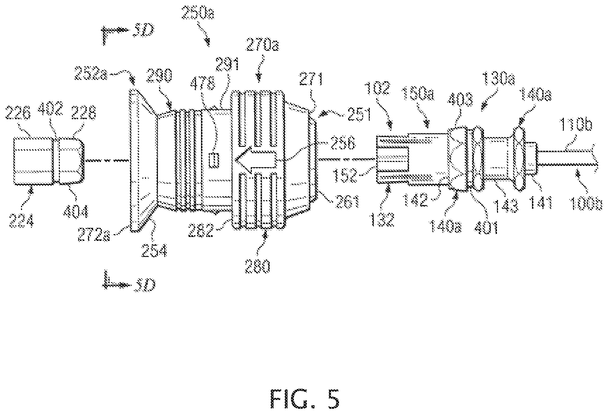

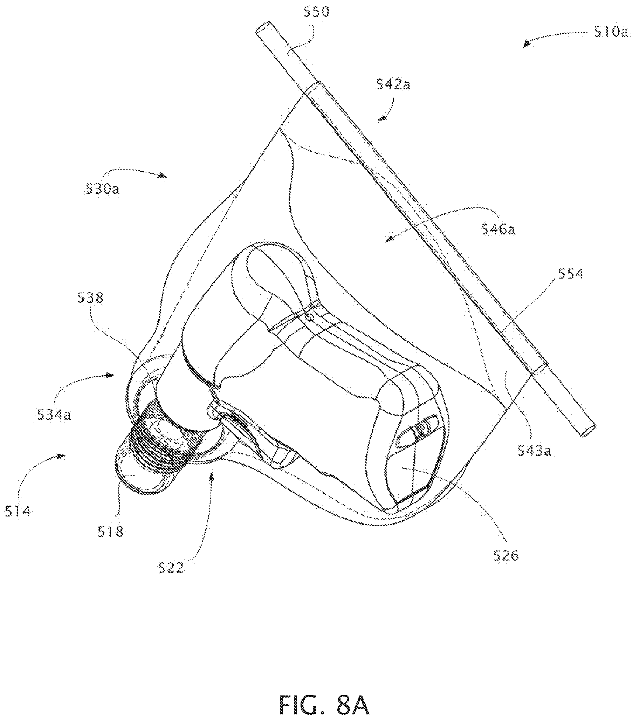

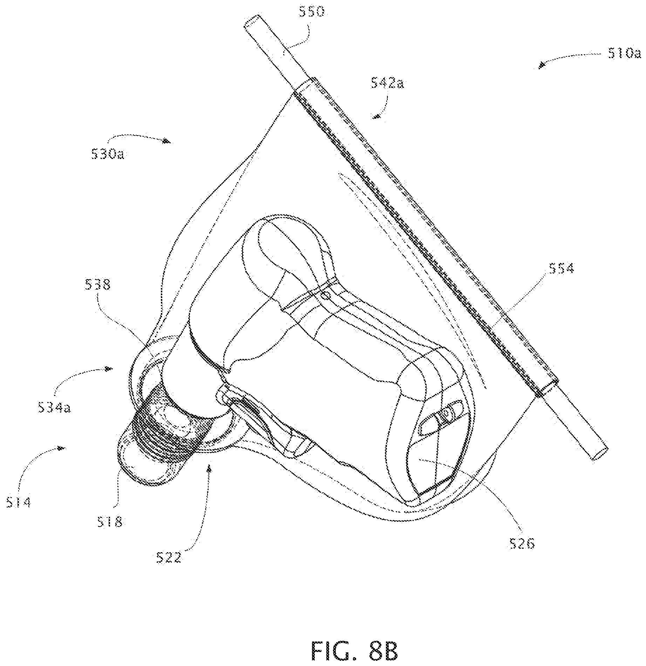

Referring now to Figs. 8A-12B, designated by the reference numerals 510a-510e are embodiments of the present containment assemblies, which are described in the claims as improved containment assemblies for drivers. In the embodiments shown, containment assemblies 510a-510e comprise coupler 514. Coupler 514 can be - but is not required to be - similar to embodiments of coupler assemblies discussed throughout this disclosure (e.g., the coupler assemblies depicted in FIGS. 5, 6A-6B, etc.). In the embodiments shown, coupler 514 comprises first end 518 and second end 522. First end 518 of coupler 514 can be configured to be coupled to intraosseous devices (e.g., such as the intraosseous devices depicted in FIGS. 1A-1D, 7A-7C, etc.). First end 518 of coupler 514 can comprise - but is not required to comprise - similar structural features to coupler assemblies discussed throughout this disclosure (such as those depicted in FIGS. 4 and 6A-6B.) to permit first end 518 of coupler 514 to be coupled to an intraosseous needle set. In other embodiments, however, first end 518 can comprise any suitable shape and/or structural configuration configured to accommodate and/or couple a given intraosseous needle set.

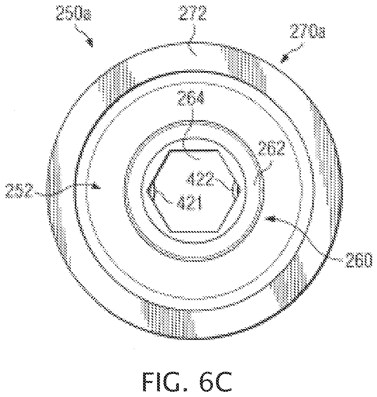

In the embodiments shown, second end 522 of coupler 514 can be configured to be coupled to a driver (e.g., a manual driver or a powered driver). For example, as depicted in FIGS. 8A-12B, second end 522 of coupler 514 is coupled to driver 526 such that driver 526 can move and/or rotate a coupler (e.g., coupler 514) and/or an intraosseous needle set, such as when an intraosseous needle set is coupled to first 518 of coupler 514. Driver 526 can be - but is not required to be - similar to embodiments of drivers discussed throughout this disclosure, such as the driver in FIG. 2. Further, second end 522 of coupler 514 can comprise - but is not required to comprise - similar structural features to coupler assemblies discussed throughout this disclosure (e.g., a hexagonal shape as depicted in FIG. 6C) to permit second end 522 of coupler 514 to be coupled to a driver. However, in other embodiments, various other embodiments of powered drivers and various other configurations of couplers can be used with containment assemblies 510a-510e.

In the embodiments shown in FIGS. 8A-11C, containment assemblies 510a-510d comprise a containment bag (e.g., containment bags 530a-530d, described collectively as containment bags 530). Embodiments of the present containment bags may comprise various materials, such as plastic, rubber, suitable elastic material (e.g., latex) configured to bias the containment bag toward a device disposed within the containment bag, and/or any generally fluid and/or microbe impervious material that can be sterilized. A containment bag (e.g., containment bags 530) can be configured to receive a non-sterile driver (e.g., manual or powered) in order to prevent the driver from contaminating a sterile intraosseous device and/or target area.

In the embodiments shown in FIGS. 8A-11C, containment bags 530 can comprise a first end (e.g., first ends 534a-534d, described collectively as first ends 534). In the embodiments shown, first ends 534 can be configured to be coupled to second end 522 of coupler 514 such that coupler 514 covers a first opening (e.g., first openings 538a-538d, described collectively as first openings 538) of containment bags 530. In the embodiments shown, second end 522 of coupler 514 can cooperate with first ends 534 of containment bags 530 to form a fluid and/or sterile seal over first openings 538. Coupler 514 can be coupled to and/or cooperate with containment bags 530 in any suitable way to cover first openings 538, such as to prevent the interior of containment bags 530 from desterilizing an intraosseous device and/or a target area. For example, in some embodiments, coupler 514 can be coupled to containment bags 530 as previously described in detail in this disclosure (e.g., via assemblies of annular rings, posts, holes, flanges, and/or the like). In some embodiments, containment bags 530 can be coupled (e.g., attached via, for example, an adhesive, sonic welds, both, or the like) to coupler 514 so that an interior (or proximal) surface of the coupler is next to an exterior surface of the containment bag. In other embodiments, containment bags 530 can be coupled (e.g., attached via, for example, an adhesive, sonic welds, both, or the like) to coupler 514 so that an exterior (or distal) surface of the coupler is next to an interior surface of the containment bag. In still other embodiments, containment bags 530 can be coupled to coupler 514 such that a portion of the coupler (e.g., second end 522) extends adjacent to both interior and exterior surfaces (e.g., on either side) of the containment bag.

In the embodiments shown in FIGS. 8A-11C, containment bags 530 also include a second end (e.g., second ends 542a-542d, collectively described as second ends 542), a second opening (e.g., second openings 546a-546d, collectively described as second openings 546), and a flap (e.g., flaps 543a-543d, collectively described as flaps 543) between the second end and the second opening. In other embodiments, the flap is positioned coincident with and extends laterally from the second opening, such that the second end of the bag is coincident with the second opening. Second ends 542 is configured (e.g., sized) to receive and/or accommodate a driver (e.g., driver 526) through second openings 546 to permit the driver to be coupled to coupler 514 (such as through first openings 538 for the type of couplers shown in FIGS. 6A and 6B). In the embodiments shown, containment bags 530 are configured to close and/or seal a driver (e.g., driver 526) in the interior of containment bags 530 (e.g., after coupling the driver to coupler 514).

The present containment assemblies depict various embodiments of closable/sealable containment bags and structural features of containment assemblies operable to close and/or seal a containment bag with a driver in the interior of the containment bag. For example, in the embodiment shown in FIGS. 8A-8B, containment assembly 510a comprises rod 550 configured to be coupled (e.g., and coupled, in the embodiment shown) to second end 542a of containment bag 530a such that second opening 546a can be closed (e.g., and/or sealed) by wrapping a portion or all of flap 543a of containment bag 530a around rod 550 (e.g., by rotating rod 550 toward first end 534a of containment bag 530a). Rod 550 can be coupled to containment bag 530a in any suitable manner to permit containment bag 530a to be wrapped around rod 550 to close second opening 546a. In some embodiments, rod 550 is coupled to flap 543a starting at or near second end 542a of containment bag 530a, and is coupled in that fashion prior to a user first using the containment bag; in other embodiments, containment assembly 510a is configured to permit a user to couple rod 550 to second end 542a before using the containment bag. For example, in some embodiments, a user can wrap a loose portion of second end 542a of containment bag 530a around rod 550 such that the user can rotate rod 550 toward first end 534a to close second opening 546a. In other embodiments, second end 542a of containment bag 530a can comprise seam 554 that defines a pouch in which rod 550 is configured to be disposed and/or coupled. In some embodiments, second end 542a of containment bag 530a can be configured such that a user can create seam 554 (e.g., by disposing an adhesive along second end 534a to enable a user to fold second end 534a onto containment bag 530a to form seam 554). In the embodiment shown, rod 550 comprises a substantially cylindrical configuration (e.g., with a circular and/or ovular cross-section). Rod 550 can be substantially rigid or flexible. In other embodiments, rod 550 can comprise any configuration suitable to be coupled to containment bag 530a (e.g., an elongated rectangle, etc.), provided the rod is adjacent to only one side of the containment bag prior to closing the second opening of the containment bag, as is the embodiment of rod 550 shown in the figures.

In some embodiments of the containment assemblies in FIGS. 8A-8B, rod 550 can be configured (e.g., hooked, sticky, etc.) and/or configurable (e.g., bendable, twistable, etc.) to prevent second end 542a of containment bag 530a from moving away from first end 534a after containment bag 530a has been wrapped around rod 550 to close second end 546a. For example, at least a portion of rod 550 can extend beyond containment bag 530a (e.g., or beyond a pouch defined by seam 554) such that the portion extending beyond containment bag 530a can be folded and/or bent toward containment bag 530a (e.g., parallel to rod 550, but not as long as the length of the portion of rod 550 that spans one side of the containment bag) to prevent containment bag 530a from moving away from first end 534a.

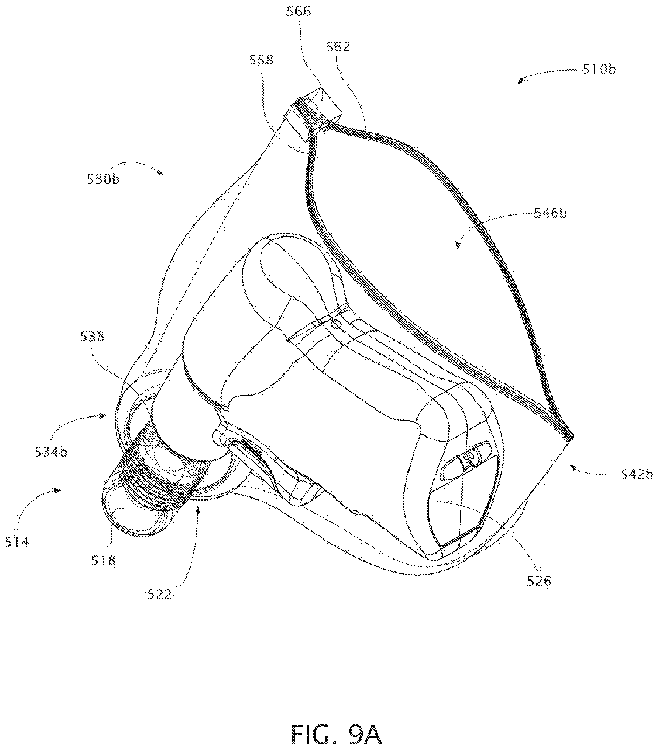

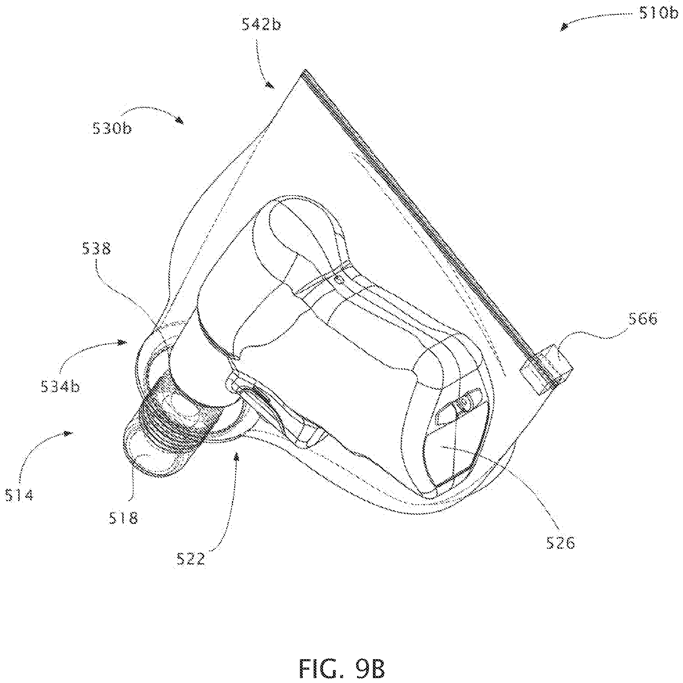

Some embodiments of the present methods, which include methods involving the use of containment assemblies (e.g., sterilized containment assemblies), include inserting a driver (e.g., driver 526) into a containment bag (e.g., containment bag 530a) of a containment assembly (e.g., containment assembly 510a), and coupling the driver to a coupler (e.g., second end 522 of coupler 514) that is coupled to the containment bag. The containment bag has an opening (e.g., second opening 546a), and it also has a rod (e.g., rod 550) that is positioned farther from the driver than is the opening. These embodiments also include rotating the rod toward the coupler to close the opening. In some more specific embodiments, the method can also include coupling an intraosseous needle set/device to the coupler (e.g., first end 518) and activating the driver to move (e.g., rotate) the intraosseous needle set/device. FIGS. 9A-9B depict another embodiment of a containment bag configured to be closable and/or sealable to prevent a driver from desterilizing an intraosseous device and/or a target area. In the embodiment shown, containment assembly 510b comprises first lockable track 558 (such as one that comprises a male member or a female member (or aspects of both) and that is similar or identical to those used for the resealing mechanism on resealable bags (e.g., for perishable goods)) extending along second end 542b of containment bag 530b adjacent to second opening 546b. Containment assembly 510b further comprises second lockable track 562 (such as one that comprises a male member or a female member (or aspects of both) and that is similar or identical to those used for the resealing mechanism on resealable bags (e.g., for perishable goods)) also extending along second end 542b adjacent to second opening 546b and opposite first lockable track 558 (e.g., such that first lockable track 558 and second lockable track 562 face one another). In some embodiments, the first and second lockable tracks may comprise zipper tracks. First lockable track 558 and second lockable track 562 can be coupled to second end 542b of containment bag 530b in any suitable manner, such as, for example, by an adhesive. In some embodiments, first and second lockable tracks 558 and 562 are unitary with containment bag 530b (e.g., formed of a single piece of material).

In the embodiment shown in FIGS. 9A-9B, containment assembly 510b further comprises tab 566 configured to be coupled (e.g., and coupled, in the embodiment shown) to first lockable track 558 and second lockable track 562. In the embodiment shown, tab 566 is configured to be moved in a first direction (e.g., along first and second lockable tracks 558 and 562 lateral to second opening 546b) to cause first lockable track 558 to engage second lockable track 562 (e.g., closing second opening 546b and/or sealing the interior of containment bag 530b). In some embodiments, tab 566 can be moved in a second direction (e.g., opposite the first direction and lateral to second opening 546b) to cause first lockable track 558 to disengage second lockable track 562 (e.g., opening second opening 546b and/or unsealing the interior of containment bag 530b). In some embodiments, first lockable track 558 can comprise teeth to engage second lockable track 562.

Some embodiments of the present exemplary methods, which include methods involving the use of containment assemblies (e.g., sterilized containment assemblies), include inserting a driver (e.g., driver 526, where the driver can be sterile or non-sterile) into a containment bag having an open end (e.g., containment bag 530b) of a containment assembly (e.g., containment assembly 510b), coupling the driver to a coupler (e.g., second end 522 of coupler 514) that is coupled to the containment bag, and moving a tab (e.g., tab 566) in a first direction to cause a first lockable track (e.g., lockable track 558) to engage a second lockable track (e.g., lockable track 562) to close an opening (e.g., second opening 546b) in the containment bag to enclose and/or seal the driver in the interior of the containment bag. In some more specific embodiments, the method can further comprise coupling an intraosseous needle set/device to the coupler and activating the driver to move the intraosseous needle set/device. In other embodiments, the method can include moving the tab in a second direction (e.g., opposite the first direction) to cause the first lockable track to disengage the second lockable track to open the opening in the containment bag (e.g., to permit a user to decouple and/or remove the driver).

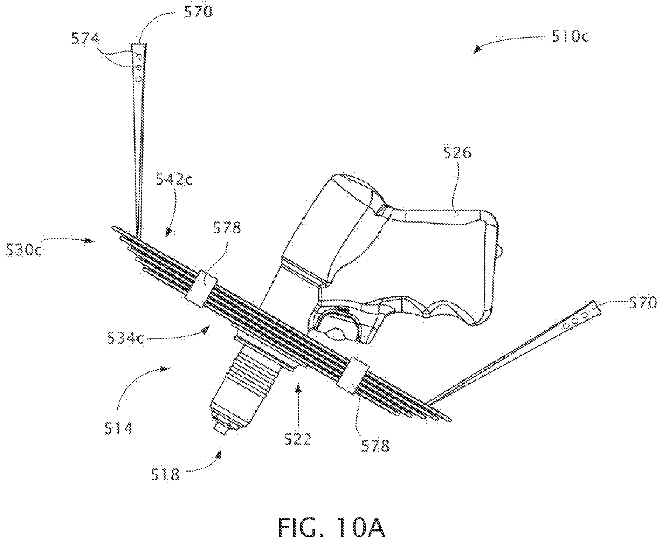

FIGS. 10A-10B depict another embodiment of a containment bag configured to be closable and/or sealable to prevent a driver from desterilizing an intraosseous device and/or a target area. In the embodiment shown, containment assembly 510c comprises at least one drawstring 570 configured to be pullable to cinch and seal the interior of containment bag 530 by closing second opening 546c of containment bag 530. In the embodiment shown, at least one drawstring 570 is coupled to and/or configured to be coupled to second end 542c of containment bag 530c. In some embodiments, at least one drawstring 570 can be unitary with second end 542c of containment bag 530c (e.g., formed of a single piece of material). At least one drawstring 570 can comprise any suitable material (e.g., plastic, nylon, and the like) to be coupled to containment bag 530c and can comprise a material similar to or different from containment bag 530c. In the embodiment shown, containment assembly 510c comprises more than one drawstring 570 configured to be tied together to close second opening 546c and/or secure second end 542c in a closed configuration. In some embodiments, at least one drawstring 570 can comprise more than one end configured to be tied together to close second opening 546c and/or secure second end 542c in a closed configuration. In the embodiment shown, at least one drawstring 570 further comprises at least one opening 574 configured to be gripped (e.g., by a user, when pulling drawstring 570 to cinch containment bag 530c). At least one opening 574 is also configured to suspend containment assembly 510c and/or a driver (e.g., driver 526) if at least one opening 574 is coupled to a supporting structure (e.g., a hook, pin, etc.).

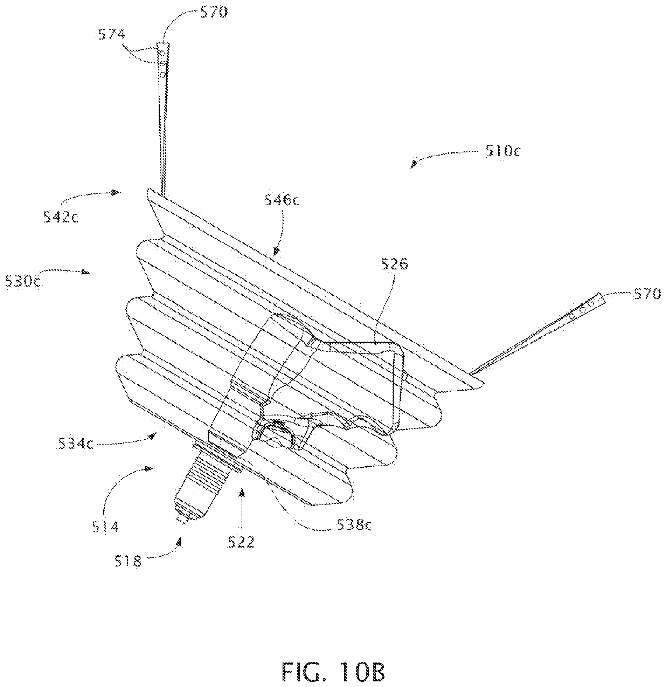

In the embodiment shown, containment bag 530c can be in a folded configuration (e.g., FIG. 10A) and an unfolded configuration (e.g., FIG. 10B). Containment bag 530c is configured such that a user can apply a sufficient force to (e.g., pull) first end 534c, second end 542c, coupler 514, and/or at least one drawstring 570 to move containment bag 530c from a folded configuration to an unfolded configuration. In the embodiment shown, containment assembly 510c further comprises at least one retaining strip 578 configured to be coupled (e.g., coupled, in the embodiment shown) to first and second ends 534c and 542c of containment bag 530c to maintain containment bag 530c in a folded configuration until a user decouples retaining strip 578. At least one retaining strip 578 is configured to decouple from first end 534c and/or second end 542c of containment bag 530c when a user applies a sufficient force to (e.g., pulls) at least one of first end 534c, second end 542c, coupler 514, and at least one drawstring 570. Retaining strip 578 can comprise any suitable pressure-sensitive adhesive (e.g., acrylics, rubbers, and similar materials). Retaining strip 578 can also comprise any other material (or combinations of materials) configured to decouple from containment bag 530c upon application of sufficient force (e.g., plastic, nylon, paper, etc.). At least one retaining strip 578 can be coupled to containment bag 530c or can be configured such that a user can couple at least one retaining strip 578 to containment bag 530c.

Some embodiments of the present exemplary methods, which include methods involving the use of containment assemblies (e.g., sterilized containment assemblies), include inserting a driver (e.g., driver 526, where the drive can be sterile or non-sterile) into a containment bag having an open end (e.g., containment bag 530c) of a containment assembly (e.g., containment assembly 510c), coupling the driver to a coupler (e.g., second end 522 of coupler 514) that is coupled to the containment bag, and pulling at least one drawstring (e.g., at least one drawstring 570) to cinch the containment bag to close an opening (e.g., second opening 546c of containment bag 530c) in order to enclose and/or seal the driver in the interior of the containment bag. In some embodiments, if the containment bag is prevented from moving to an unfolded configuration by at least one retaining strip (e.g., at least one retaining strip 578) coupled to the containment bag, then the method can include applying a sufficient force to decouple the at least one retaining strip from the containment bag to permit the containment bag to move into the unfolded configuration. In some embodiments, the method can include tying two ends of the at least one drawstring (e.g., when the at least one drawstring has two ends) and/or tying two or more drawstrings (e.g., when more than one drawstring is coupled to the containment bag) together to keep the opening of the containment bag closed. In some more specific embodiments, the method can further comprise coupling an intraosseous needle set/device to the coupler and activating the driver to move the intraosseous needle set/device.

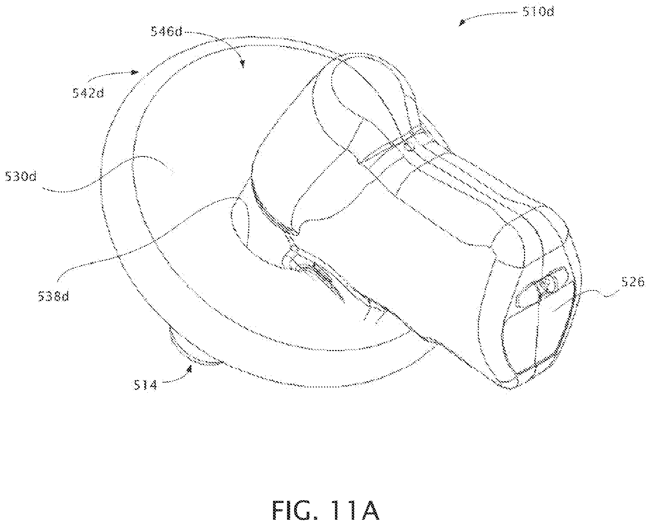

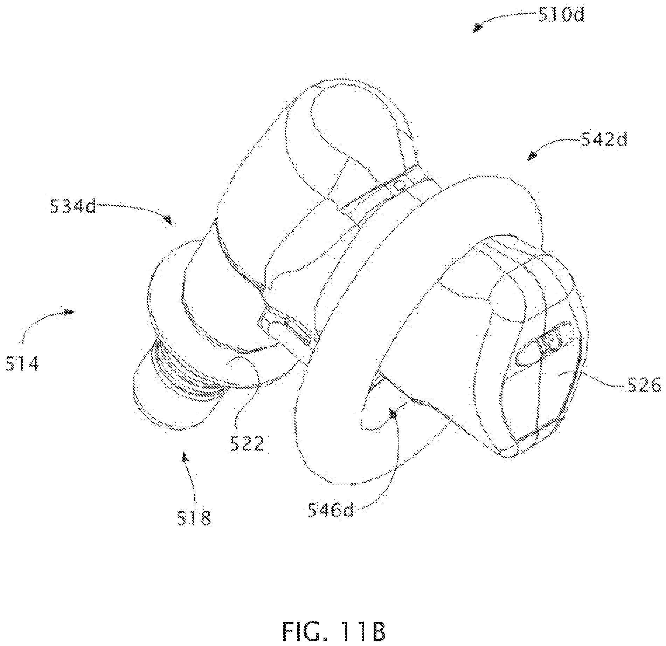

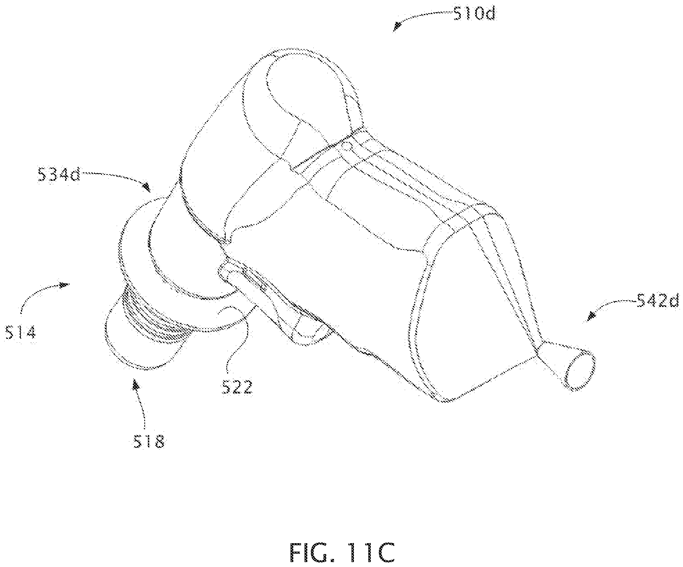

FIGS. 11A-11C depict another embodiment of a containment bag configured to be closable and/or sealable to prevent a driver from desterilizing an intraosseous device and/or a target area. In the embodiment shown, containment assembly 510d is configured such that containment bag 530d can be in a rolled configuration (e.g., FIG. 11A) and an unrolled configuration (FIG. 11C). Second end 542d of containment bag 530d is configured to be rolled away (e.g., by a user) from first end 534d over a driver (e.g., driver 526) into an unrolled configuration such that the driver is within the interior of containment bag 530d. In the embodiment shown, containment bag 530d is configured such that when containment bag 530d is in an unrolled configuration, second end 542d of containment bag 530d extends beyond the driver such that second end 542d can be tied to close second opening 546d and seal the driver in the interior of containment bag 530d. In the embodiment shown, containment bag 530d is configured to be biased radially inward toward a driver (e.g., driver 526) such that containment bag 530d substantially forms to the shape of the driver, and containment bag 530d can comprise any suitable material to do so (e.g., such as an elastic material (e.g., latex)).

Some embodiments of the present exemplary methods, which include methods involving the use of containment assemblies (e.g., sterilized containment assemblies), include inserting a driver (e.g., driver 526, where the driver can be sterile or non-sterile) into a containment bag having an open end (e.g., containment bag 530d) of a containment assembly (e.g., containment assembly 510d), coupling the driver to a coupler (e.g., second end 522 of coupler 514) that is coupled to the containment bag, rolling the containment bag to move the containment bag to an unrolled configuration (e.g., by rolling second end 542d of containment bag 530d away from first end 534d), and closing the open end of the containment bag. In some embodiments, the method can include tying the containment bag (e.g., second end 542d of containment bag 530d) to seal the driver in the interior of the containment bag. In some more specific embodiments, the method can include coupling an intraosseous needle set/device to the coupler (e.g., first end 518 of coupler 514) and activating the driver to move the intraosseous needle set/device.

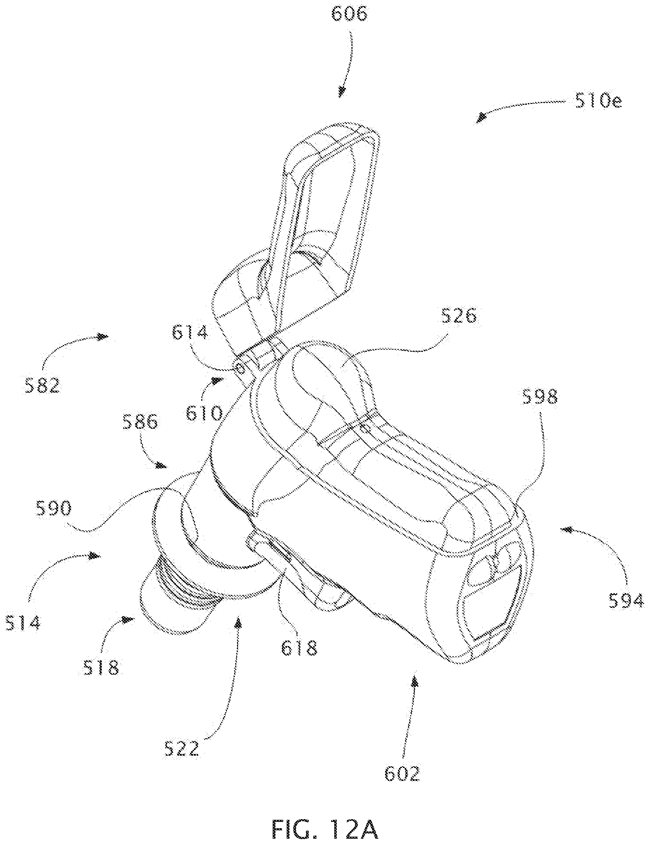

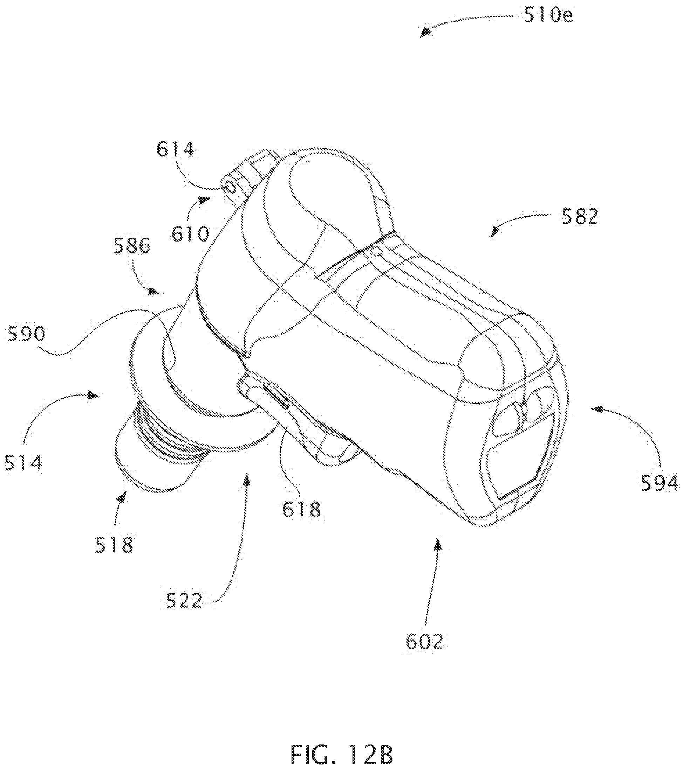

In addition to containment bags, the present containment assemblies can also include substantially rigid casings. A substantially rigid casing - similarly to a containment bag - can be configured to prevent a driver from desterilizing a sterile intraosseous device and/or target area. For example, in the embodiment, in accordance with the present claimed invention, shown in FIGS. 12A-12B, containment assembly 510e comprises substantially rigid casing 582. Embodiments of substantially rigid casings can comprise various shapes and configurations to accommodate a device (e.g., a driver) within the containment assembly and to enclose and/or seal the device in the interior of the substantially rigid casing. For example, in the embodiment shown, substantially rigid casing 582 comprises a substantially driver-shaped configuration (e.g., having a housing with a handle). Substantially rigid casing 582 can comprise - but is not required to comprise - similar materials to containment bags as described throughout this disclosure, such as rubber, plastic (e.g., thermosets, thermoplastics, polyvinyl chloride, Teflon, polymers, polycarbonate, and the like), and any generally fluid and/or microbe impervious rigid material that can be sterilized.

In the embodiment shown in FIGS. 12A-12B, substantially rigid casing 582 comprises first end 586 coupled to second end 522 of coupler 514 such that coupler 514 covers first opening 590 of substantially rigid casing 582. In the embodiment shown, second end 522 of coupler 514 can cooperate with first end 586 of substantially rigid casing 582 to form a fluid and/or sterile seal over first opening 590. Coupler 514 can be coupled to and/or cooperate with substantially rigid casing 582 in any way suitable to cover first opening 590 (e.g., to prevent desterilization of an intraosseous device and/or a target area). For example, in some embodiments, coupler 514 can be coupled to substantially rigid casing 582 similarly to a containment bag as described in this disclosure (e.g., via assemblies of annular rings, posts, holes, flanges, adhesives, Velcro, coupling devices, and/or the like). In some embodiments, first end 586 of substantially rigid casing 582 can comprise a female configuration and second end 522 of coupler 514 can comprise a corresponding male configuration (e.g., or vice versa). In other embodiments, second end 522 of coupler 514 can comprise male threads that correspond to female threads on first end 586 of substantially rigid casing 582 (e.g., or vice versa). In some embodiments, substantially rigid casing 582 can be coupled (e.g., attached via, for example, an adhesive, sonic welds, both, or the like) to coupler 514 so that an interior (or proximal) surface of the coupler is next to an exterior surface of the substantially rigid casing. In other embodiments, substantially rigid casing 582 can be coupled (e.g., attached via, for example, an adhesive, sonic welds, both, or the like) to coupler 514 so that an exterior (or distal) surface of the coupler is next to an interior surface of the containment bag. In still other embodiments, substantially rigid casing 582 can be coupled to coupler 514 such that a portion of the coupler (e.g., second end 522) extends adjacent to both interior and exterior surfaces (e.g., on either side) of the substantially rigid casing. In accordance with the present claimed invention substantially rigid casing 582 is couplable and decouplable to second end 522 of coupler 514 such that a user can couple substantially rigid casing 582 to coupler 514 prior to use and decouple substantially rigid casing 582 after use.

In the embodiment shown in FIGS. 12A-12B, second end 522 of coupler 514 can be configured to be coupled to a driver (e.g., a manual driver or a powered driver). For example, as depicted in FIGS. 8A-12B, second end 522 of coupler 514 is coupled to driver 526 such that driver 526 can move and/or rotate a coupler (e.g., coupler 514) and/or an intraosseous needle set, such as when an intraosseous needle set is coupled to first 518 of coupler 514. Driver 526 can be - but is not required to be - similar to embodiments of drivers discussed throughout this disclosure, such as the driver in FIG. 2. Further, second end 522 of coupler 514 can comprise - but is not required to comprise - similar structural features to coupler assemblies discussed throughout this disclosure (e.g., a hexagonal shape as depicted in FIG. 6C) to permit second end 522 of coupler 514 to be coupled to a driver. However, in other embodiments, various other embodiments of powered drivers and various other configurations of couplers can be used with containment assembly 510e..

In the embodiments shown in FIGS. 12A-12B, substantially rigid casing 582 further comprises second end 594 having second opening 598. Second end 594 can be configured to receive and/or accommodate a driver (e.g., driver 526) through second opening 598 to permit the driver to be coupled to coupler 514. In the embodiments shown, second end 594 of substantially rigid casing 582 is further configured to close second opening 598 and/or seal a driver (e.g., driver 526) in the interior of substantially rigid casing 582 (e.g., after coupling the driver to coupler 514).

In the embodiment shown in FIGS. 12A-12B, substantially rigid casing 582 comprises front portion 602. Front portion 602 comprises first end 586 (e.g., having first opening 590) and second end 594 (e.g., having second opening 598). In the embodiment shown, front portion 602 comprises a majority (e.g., 51-95% or more) of substantially rigid casing 582. In other embodiments, however, front portion 602 can comprise a minority (e.g., 5-49% or less) of substantially rigid casing 582. In the embodiment shown, front portion 602 is configured to receive and/or accommodate a driver (e.g., driver 526) through second opening 598 to permit the driver to be coupled to coupler 514. In the embodiment shown, containment assembly 510 also comprises back portion 606 coupled (e.g., movably) to front portion 602. In the embodiment shown, back portion 606 comprises a minority (e.g., 5-49%, or less) of substantially rigid casing. However, in other embodiments, back portion 606 can comprise a majority of substantially rigid casing (e.g., 51-95%, or more) such that front portion and/or another portion comprises a minority of substantially rigid casing). In other embodiments, front portion 602 and back portion 606 can comprise substantially equal portions (e.g., 50%) of substantially rigid casing 582. Back portion 606 is configured to cooperate with front portion 602 to accommodate a driver (e.g., driver 526). For example, back portion 606 and front portion 602 can cooperate to define a substantially driver-shaped (e.g., as in the embodiment shown). Back portion 606 can cooperate with front portion 602 to substantially enclose the driver within the chamber and/or seal the driver in the interior of substantially rigid casing 582.

In the embodiment shown in FIGS. 12A-12B, back portion 606 is coupled (e.g., rotatably) to front portion 602 by hinge 610. In the embodiment shown, hinge 610 has hinge pin 614 configured to permit rotational motion of back portion 606 with respect to front portion 602. In other embodiments, back portion 606 can be coupled to front portion 602 in various ways such as, for example, with a living hinge, a bearing, a barrel hinge, and the like. In some embodiments, back portion 606 can be configured to be detachably coupled to front portion 602 such that a user can couple and decouple back portion 606 from front portion 602.

In the embodiment shown in FIGS. 12A-12B, substantially rigid casing 582 further comprises and/or is coupled to flexible membrane 618. In the embodiment shown, flexible member 618 is configured to permit a user to engage a trigger of a driver (e.g., driver 526) when the driver is disposed in substantially rigid casing 582. Flexible member 618 can comprise, for example, any suitable material through which a user can engage a trigger, such as, elastomeric material, rubber, latex, and the like. Flexible membrane 618 can also be disposed and/or coupled to any other portion of a driver to allow a user to engage that portion of the driver through substantially rigid casing 582 (e.g., to change a power source, to engage another button, etc.).

Some embodiments of the present exemplary methods, which include methods involving the use of containment assemblies (e.g., sterilized containment assemblies), include inserting a driver (e.g., driver 526, where the driver can be sterile or non-sterile) into a front portion (e.g., front portion 602) of substantially rigid casing (e.g., substantially rigid casing 582) of a containment assembly (e.g., containment assembly 510e), coupling the driver to a coupler (e.g., second end 522 of coupler 514) that is coupled to the substantially rigid casing, and closing a back portion (e.g., back portion 606) of the substantially rigid casing such that the back portion cooperates with a front portion (e.g., front portion 602) to substantially enclose the driver. In some embodiments, the method can include coupling an intraosseous needle set/device to the coupler and activating the driver to move the intraosseous needle set.

Similarly to other embodiments of intraosseous devices (or components of intraosseous devices) described in this disclosure, embodiments of the present containment assemblies (and components of such embodiments) can also be included in one or more kits. A kit containing one or more embodiments (or one or more components) of the present containment assemblies can comprise one or more IO devices (or one or more components of IO devices) of any of the kits described in this disclosure (e.g., as depicted in FIG. 7A-7C). For example, a kit can include any one of containment assemblies 510a-510e. A kit can further include an intraosseous needle set/device (e.g., a stylet, a cannula, and the like) configured to be coupled to a coupler (e.g., coupler 514), a driver configured to be coupled to a coupler (e.g., coupler 514), a fluid bag (e.g., configured to administer IV fluids), an aspiration device (e.g., configured to aspirate tissue, bone, and/or fluid from a target area), a sharps protector, and similar devices described and depicted throughout this disclosure.

The above specification and examples provide a complete description of the structure and use of exemplary embodiments. Although certain embodiments have been described above with a certain degree of particularity, or with reference to one or more individual embodiments, those skilled in the art could make numerous alterations to the disclosed embodiments without departing from the scope of this invention as defined by the following claims. As such, the various illustrative embodiments of the present devices are not intended to be limited to the particular forms disclosed.

## Claims

1. A containment assembly for a driver, the containment assembly comprising:
a coupler (514) having a first end (518) configured to be coupled to an intraosseous needle set and a second end (522) configured to be coupled to a driver (526) such that the driver (526) can move the intraosseous needle set; and
a substantially rigid casing (582) comprising:
a front portion (602) having a first opening (590) and a second opening (598), the front portion (602) coupled to the coupler (514) such that the coupler (514) covers the first opening (590), and the second opening (598) configured to receive the driver (526) such that the driver (526) can be coupled to the coupler (514); and
a back portion (606) movably coupled to the front portion (602), the back portion (606) configured to cooperate with the front portion (602) to accommodate the driver (526) that is coupled to the coupler (514),
wherein the substantially rigid casing (582) can be coupled to and decoupled from the second end (522) of the coupler (514).

2. The containment assembly of claim 1, where the casing (582) is substantially sterile.

3. The containment assembly of any one of claims 1 and 2, where the front portion (602) and the back portion (606) of the casing (582) cooperate, when coupled together, to define a driver-shaped chamber.

4. The containment assembly of any one of claims 1, 2, and 3, where the back portion (606) is rotatably coupled to the front portion (602) by a hinge pin (614).

5. The containment assembly of any one of claims 1, 2, and 3, where the back portion (606) is rotatably coupled to the front portion (602) by a living hinge.

6. The containment assembly of any one of claims 1, 2, and 3, where the back portion (606) is detachably coupled to the front portion (602).

7. The containment assembly of claim 1, where the front portion (602) of the casing (582) comprises a flexible membrane (618) configured to permit a user to engage a trigger of the driver (526) that is disposed in the casing (582).

8. The containment assembly of claim 1, where a portion of the second end (522) of the coupler (514) extends adjacent to an interior surface and an exterior surface of the substantially rigid casing (582) when the front portion (602) of the substantially rigid casing (582) is coupled to the coupler (514).

9. The containment assembly of claim 1, where the front portion (602) and the back portion (606) of the substantially rigid casing (582) can cooperate to seal the driver (526) in an interior of the substantially rigid casing (582).

10. The containment assembly of claim 9, where the driver (526) comprises a housing and a motor, where the motor is disposed within portions of the housing.

11. The containment assembly of claim 1, where the substantially rigid casing (582) comprises a substantially driver-shaped configuration.

12. The containment assembly of claim 1, where the substantially rigid casing (582) comprises a flexible membrane (618) configured to allow a user to change a power source of the driver (526).

13. The containment assembly of claim 1, where the front portion (602) of the substantially rigid casing (582) comprises a greater portion of the substantially rigid casing (582) than the back portion (606) of the substantially rigid casing (582).

## Patentansprüche

1. Einschlussanordnung für einen Antrieb, bei welcher die Einschlussanordnung Folgendes beinhaltet:
einen Koppler (514), welcher ein erstes Ende (518) aufweist, welches konfiguriert ist, um mit einem intraossären Nadelset gekoppelt zu werden und ein zweites Ende (522), welches konfiguriert ist, um mit einem Antrieb (526) gekoppelt zu werden, so dass der Antrieb (526) das intraossäre Nadelset bewegen kann; und
ein im wesentlichen starres Gehäuse (582), Folgendes beinhaltend:
einen vorderen Abschnitt (602), welcher eine erste Öffnung (590) und eine zweite Öffnung (598) aufweist, wobei der vordere Abschnitt (602) mit dem Koppler (514) in einer Weise gekoppelt ist, dass der Koppler (514) die erste Öffnung (590) bedeckt, und die zweite Öffnung (598) konfiguriert ist, um den Antrieb (526) in einer Weise aufzunehmen, dass der Antrieb (526) mit dem Koppler (514) gekoppelt werden kann; und
einen hinteren Abschnitt (606), welcher beweglich mit dem vorderen Abschnitt (602) gekoppelt ist, wobei der hintere Abschnitt (606) konfiguriert ist, um mit dem vorderen Abschnitt (602) zusammenzuarbeiten, um den Antrieb (526) aufzunehmen, welcher mit dem Koppler (514) gekoppelt ist,
wobei das im Wesentlichen starre Gehäuse (582) mit dem zweiten Ende (522) des Kopplers (514) gekoppelt und davon entkoppelt werden kann.

2. Einschlussanordnung nach Anspruch 1, bei welcher das Gehäuse (582) im Wesentlichen steril ist.

3. Einschlussanordnung nach einem der Ansprüche 1 und 2, bei welcher der vordere Abschnitt (602) und der hintere Abschnitt (606) des Gehäuses (582) zusammenarbeiten, wenn sie miteinander gekoppelt sind, um eine antriebsförmige Kammer zu definieren.

4. Einschlussanordnung nach einem der Ansprüche 1, 2 und 3, bei welcher der hintere Abschnitt (606) drehbar mit dem vorderen Abschnitt (602) durch einen Scharnierstift (614) gekoppelt ist.

5. Einschlussanordnung nach einem der Ansprüche 1, 2 und 3, bei welcher der hintere Abschnitt (606) drehbar mit dem vorderen Abschnitt (602) durch ein bewegliches Scharnier gekoppelt ist.

6. Einschlussanordnung nach einem der Ansprüche 1, 2 und 3, bei welcher der hintere Abschnitt (606) abnehmbar mit dem vorderen Abschnitt (602) gekoppelt ist.

7. Einschlussanordnung nach Anspruch 1, bei welcher der vordere Abschnitt (602) des Gehäuses (582) eine flexible Membran (618) beinhaltet, welche konfiguriert ist, um es einem Bediener zu ermöglichen, in einen Auslöser des Antriebs (526), welcher in dem Gehäuse (582) angeordnet ist, einzugreifen.

8. Einschlussanordnung nach Anspruch 1, bei welcher ein Abschnitt des zweiten Endes (522) des Kopplers (514) sich angrenzend an eine innere Fläche und an eine äußere Fläche des im Wesentlichen starren Gehäuses (582) erstreckt, wenn der vordere Abschnitt (602) des im Wesentlichen starren Gehäuses (582) mit dem Koppler (514) gekoppelt ist.

9. Einschlussanordnung nach Anspruch 1, bei welcher der vordere Abschnitt (602) und der hintere Abschnitt (606) des im Wesentlichen starren Gehäuses (582) miteinander zusammenarbeiten können, um den Antrieb (526) in einem Inneren des im Wesentlichen starren Gehäuses (582) dicht einzuschließen.

10. Einschlussanordnung nach Anspruch 9, bei welcher der Antrieb (526) eine Einhausung und einen Motor beinhaltet, wobei der Motor innerhalb von Abschnitten der Einhausung angeordnet ist.

11. Einschlussanordnung nach Anspruch 1, bei welcher das im Wesentlichen starre Gehäuse (582) eine im Wesentlichen antriebsförmige Konfiguration beinhaltet.

12. Einschlussanordnung nach Anspruch 1, bei welcher das im Wesentlichen starre Gehäuse (582) eine flexible Membran (618) beinhaltet, welche konfiguriert ist, um den Benutzer in die Lage zu versetzen, eine Stromquelle des Antriebs (526) auszuwechseln.

13. Einschlussanordnung nach Anspruch 1, bei welcher der vordere Abschnitt (602) des im Wesentlichen starren Gehäuses (582) einen größeren Abschnitt des im Wesentlichen starren Gehäuses (582) als der hintere Abschnitt (606) des im Wesentlichen starren Gehäuses (582) beinhaltet.

## Revendications

1. Ensemble de confinement pour un moyen d'entraînement, l'ensemble de confinement comprenant :
un coupleur (54) comportant une première extrémité (518) configurée pour être accouplée à un set d'aiguilles intra-osseuses, et une deuxième extrémité (522) configurée pour être accouplée à un moyen d'entraînement (526), de sorte que le moyen d'entraînement (526) peut déplacer le set d'aiguilles intra-osseuses ; et
un logement sensiblement rigide (582) comprenant :
une partie avant (602) comportant une première ouverture (590) et une deuxième ouverture (598), la partie avant (602) étant accouplée au coupleur (514) de sorte que le coupleur (514) recouvre la première ouverture (590), la deuxième ouverture (598) étant configurée pour recevoir le moyen d'entraînement (526), de sorte que le moyen d'entraînement (526) peut être accouplé au coupleur (514) ; et
une partie arrière (606) accouplée de manière mobile à la partie avant (602), la partie arrière (606) étant configurée pour coopérer avec la partie avant (602) pour recevoir le moyen d'entraînement (526) accouplé au coupleur (514) ;
dans lequel le logement sensiblement rigide (582) peut être accouplé à la deuxième extrémité (522) du coupleur (514) et désaccouplé de celle-ci.

2. Ensemble de confinement selon la revendication 1, dans lequel le logement (582) est sensiblement stérile.

3. Ensemble de confinement selon l'une quelconque des revendications 1 et 2, dans lequel la partie avant (602) et la partie arrière (606) du logement (582) coopèrent, lors de leur accouplement, pour définir une chambre en forme de moyen d'entraînement.

4. Ensemble de confinement selon l'une quelconque des revendications 1, 2 et 3, dans lequel la partie arrière (606) est accouplée de manière rotative à la partie avant (602) par une broche de charnière (614).

5. Ensemble de confinement selon l'une quelconque des revendications 1, 2 et 3, dans lequel la partie arrière (606) est accouplée de manière rotative à la partie avant (602) par une charnière vivante.

6. Ensemble de confinement selon l'une quelconque des revendications 1, 2 et 3, dans lequel la partie arrière (606) est accouplée de manière détachable à la partie avant (602).

7. Ensemble de confinement selon la revendication 1, dans lequel la partie avant (602) du logement (582) comprend une membrane flexible (618) configurée pour permettre à un utilisateur de se mettre en prise avec une gâchette du moyen d'entraînement (526) disposée dans le logement (582).

8. Ensemble de confinement selon la revendication 1, dans lequel une partie de la deuxième extrémité (522) du coupleur (514) s'étend de manière adjacente à une surface interne et à une surface externe du logement sensiblement rigide (582) lorsque la partie avant (602) du logement sensiblement rigide (582) est accouplée au coupleur (514).

9. Ensemble de confinement selon la revendication 1, dans lequel la partie avant (602) et la partie arrière (606) du logement sensiblement rigide (582) peuvent coopérer pour sceller le moyen d'entraînement (526) dans un intérieur du logement sensiblement rigide (582).

10. Ensemble de confinement selon la revendication 9, dans lequel le moyen d'entraînement (526) comprend un boîtier et un moteur, le moteur étant disposé dans des parties du boîtier.

11. Ensemble de confinement selon la revendication 1, dans lequel le logement sensiblement rigide (582) comprend une configuration sensiblement en forme de moyen d'entraînement.

12. Ensemble de confinement selon la revendication 1, dans lequel le logement sensiblement rigide (582) comprend une membrane flexible (618) configurée pour permettre à un utilisateur de changer une source d'alimentation du moyen d'entraînement (526).

13. Ensemble de confinement selon la revendication 1, dans lequel la partie avant (602) du logement sensiblement rigide (582) comprend une partie du logement sensiblement rigide (582) plus grande que la partie arrière (606) du logement sensiblement rigide (582).
